# EUROPEAN PATENT APPLICATION

(11) **EP 3 287 120 A1**
(43) Date of publication of application: **28.02.2018**
(21) Application number: 16185500.2
(22) Date of filing: 24.08.2016
(51) Int. Cl.: A61K 8/36, A61K 8/365, A61K 8/41, A61K 8/44, A61K 8/45, A61Q 5/00

(54) **METHOD FOR COLORING HAIR**

(71) Applicant: Noxell Corporation, Hunt Valley, Maryland 21030-2098 (US)
(72) Inventor: Flohr, Andreas, 65824 Schwalbach am Taunus (DE)
(74) Representative: Zimmermann & Partner Patentanwälte mbB

(57) **Abstract**

A method for coloring hair, and a kit for coloring hair are provided. The method for coloring hair comprises applying to the hair a first composition comprising in a cosmetically acceptable carrier, one or more ionizable linkers of Formula I, or cosmetically acceptable salts thereof, or mixtures thereof: 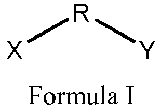 and applying to the hair a second composition comprising in a cosmetically acceptable carrier, one or more ingredients of Formula II and/or Formula III, or cosmetically acceptable salts thereof, or mixtures thereof: 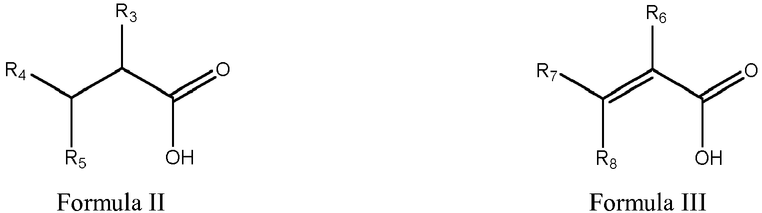 and wherein the first composition does not comprise any ingredients of Formula II and/or Formula III of the second composition and any oxidative dye precursors; wherein the second composition does not comprise any ionizable linkers of Formula I of the first composition; and wherein the second composition further comprises a dye composition and optionally a developer composition wherein the developer composition comprises one or more oxidizing agents; and wherein the dye composition comprises one or more oxidative dye precursors comprising one or more couplers and one or more primary intermediates.

## Description

### FIELD OF THE INVENTION

A method for coloring hair is provided and comprises applying to the hair a first composition comprising in a cosmetically acceptable carrier, one or more ionizable linkers of Formula I; and applying to the hair a second composition comprising in a cosmetically acceptable carrier, one or more specific ingredients of Formula II and/or of Formula III combined with a dye composition. Also, a kit for coloring hair is provided and comprises the first composition, the second composition and the dye composition which are separately packaged.

### BACKGROUND OF THE INVENTION

Hair coloring or dyeing involves the application of one or more hair dyes onto hair which results in the coloration of hair fibers. The total head of hair color may be changed subtly or dramatically, the root growth colored to match the remaining head of hair, effects introduced such as glitter, hair strand effects or other sectional effects, or the same color "freshened up" to combat fade and/or wash-out.

There is a relative high interest for some clients to get their hair turned super blonde, namely blonde platinum. However, these clients have typically a very dark hair. In order to provide the super blonde color, the dark hair needs to be bleached several times. If the hair is already heavily stressed or damaged due to previous bleaching, coloring or dying hair, such bleaching processes are not recommended. The integrity and the healthiness of the client's hair and scalp need always to be preserved and even more improved.

In that respect, some binding agents have been recently developed, see for instance International Patent Application WO 2015/017768 A1. Also, certain carboxylic acids have been used as an active ingredient for restructuring keratin fibers in cosmetics, see for instance European Patent EP 1 326 577 B2.

However, there is still a need to provide a method for coloring hair while improving the integrity and the healthiness of hair when the hair is exposed to relatively heavy stress such as bleaching and dyeing processes, i.e. processes involving oxidizing agents and oxidative dye precursors.

### SUMMARY OF THE INVENTION

The present invention is related to method for coloring hair which comprises:
(a) applying to the hair a first composition comprising in a cosmetically acceptable carrier, one or more ionizable linkers of Formula I, or cosmetically acceptable salts thereof, or mixtures thereof: 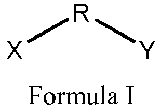
   wherein the R group of the one or more ionizable linkers is an alkyl group, alkenyl group, cycloalkyl group, cycloalkenyl group, aryl group, heterocyloalkyl group, or hetereoaryl group;
   wherein the alkyl group, alkenyl group, cycloalkyl group, cycloalkenyl group, aryl group, heterocyloalkyl group, or hetereoaryl group is unsubstituted or substituted one or more times by hydrogen, halogen, hydroxy, alkyl, alkenyl, alkynyl, alkoxy, cycloalkyl, cycloalkenyl, cycloalkynyl, aryl, heterocyclyl, heteroaryl, imine, amine, formyl, acyl, carboxylic acid, -C(O)R¹, -C(O)OR¹, (-COO-), -CONH₂, -CONHR¹,-C(O)NR¹R², -NR¹R², -NR¹S(O)₂R², -NR¹C(O)R², -S(O)₂R², -SR¹, -S(O)₂NR¹R²,-SOR¹, or -SOOR¹ and mixtures thereof; and
   wherein the one or more ionizable linkers comprises two ionizable functional groups X and Y; wherein the one or more ionizable functional groups X, Y are independently selected from the group consisting of: -C(O)NR¹R², -NR¹R², -NR¹S(O)₂R²,-NR¹C(O)R²;
   wherein R¹ and R² are each independently selected from the group consisting of a hydrogen, alkyl, alkenyl, alkynyl, cycloalkyl, cycloalkenyl, cycloalkynyl, aryl, heterocyclyl, and heteroaryl group;
(b) applying to the hair a second composition comprising in a cosmetically acceptable carrier, one or more ingredients of Formula II, or cosmetically acceptable salts thereof, or mixtures thereof: 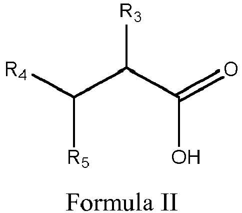
   wherein R₃ is independently selected from the group consisting of: -H, -OH, oxo,-H₂N, -C₁₋₆ alkyl groups, -C₁₋₆ alkoxy groups, -C₁₋₆ alkenyl groups;
   wherein R₄ is independently selected from the group consisting of: -H, -OH, oxo,-H₂N, -C₁₋₆ alkyl groups, -C₁₋₆ alkoxy groups, -C₁₋₆ alkenyl groups;
   wherein R₅ is independently selected from the group consisting of: -H, -C₁₋₆ alkyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid, and ionizable functional groups;
   wherein the ionizable functional group is independently selected from the group consisting of: -COOH, -SO₃H, -PO₃H₂; and/or
   wherein the second composition comprises in a cosmetically acceptable carrier, one or more ingredients of Formula III, or cosmetically acceptable salts thereof, or mixtures thereof: 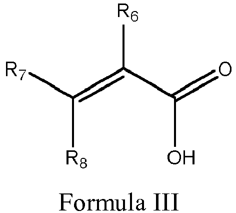
      wherein R₆ is independently selected from the group consisting of: -H, -C₁₋₆ alkyl groups, aryl groups, and ionizable functional groups;
      wherein R₇ is independently selected from the group consisting of: -H, -C₁₋₆ alkyl groups, aryl groups, and ionizable functional groups;
      wherein R₈ is independently selected from the group consisting of: -H, -C₁₋₆ alkyl groups, -C₁₋₆ alkenyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid; and ionizable functional groups;
      wherein the ionizable functional group is independently selected from the group consisting of: -COOH, -CH₂COOH, -SO₃H, -PO₃H₂.

The first composition does not comprise any ingredients of Formula II and/or Formula III of the second composition and any oxidative dye precursors. The second composition does not comprise any ionizable linkers of Formula I of the first composition. The second composition further comprises a dye composition and optionally a developer composition. The developer composition comprises one or more oxidizing agents. The dye composition comprises one or more oxidative dye precursors comprising one or more couplers and one or more primary intermediates.

The step (b) may preferably occur prior to step (a).

A kit for coloring hair is also provided and comprises:
(a) a first composition comprising in a cosmetically acceptable carrier, one or more ionizable linkers of Formula I as set out hereinbefore;
(b) a second composition comprising in a cosmetically acceptable carrier, one or more ingredients of Formula II and/or Formula III as set out hereinbefore;
   wherein the first composition does not comprise any ingredients of Formula II and/or III of the second composition and any oxidative dye precursors;
   wherein the second composition does not comprise any ionizable linkers of Formula I of the first composition; and
(c) a dye composition comprising one or more oxidative dye precursors;
(d) optionally, a developer composition comprising one or more oxidizing agents;
   wherein the first composition, the second composition, the dye composition (c) and optionally the developer composition (d) are separately packaged in different containers or packaged in a same container in different compartments; and
(e) an instructional material for preparation, for use, or both preparation and use, of the first composition, the second composition, the dye composition and optionally the developer composition.

The dye composition may be mixed with the second composition, optionally with the developer composition.

### DETAILED DESCRIPTION OF THE INVENTION

### Definitions of terms

In this document, including in all embodiments of all aspects of the present invention, the following definitions apply unless specifically stated otherwise.

All percentages are by weight (w/w) of the first composition and/or the second composition, unless otherwise specified. All ratios are weight ratios. "% wt." means percentage by weight. References to 'parts' e.g. a mixture of 1 part X and 3 parts Y, is a ratio by weight. When more than one composition are used during a treatment, the total weight to be considered is the total weight of all the compositions applied on the hair simultaneously (i.e. the weight found "on head"), typically resulting from mixing an oxidative composition (also called developer and/or oxidizing composition/component) with a dye composition (also called tint, and/or dye composition/component), unless otherwise specified. All ratios or percentages are weight ratios or weight percentages unless specifically stated otherwise.

"QS" or "QSP" means sufficient quantity for 100% or for 100g. +/- indicates the standard deviation. All ranges are inclusive and combinable. The number of significant digits conveys neither a limitation on the indicated amounts nor on the accuracy of the measurements. All numerical amounts are understood to be modified by the word "about".

All measurements are understood to be made at 20°C and at ambient conditions, where "ambient conditions" means at 1 atmosphere (atm) of pressure and at 65% relative humidity, unless otherwise stated. "Relative humidity" refers to the ratio (stated as a percent) of the moisture content of air compared to the saturated moisture level at the same temperature and pressure. Relative humidity can be measured with a hygrometer, in particular with a probe hygrometer from VWR^{®} International.

Herein "min" means "minute" or "minutes". Herein "mol" means mole. Herein "g" following a number means "gram" or "grams". "Ex." means "example". All amounts as they pertain to listed ingredients are based on the active level ('solids') and do not include carriers or by-products that may be included in commercially available materials.

Herein, "comprising" means that other steps and other ingredients can be in addition. "Comprising" encompasses the terms "consisting of" and "consisting essentially of". The compositions, methods, uses, kits, and processes of the present invention can comprise, consist of, and consist essentially of the elements and limitations of the invention described herein, as well as any of the additional or optional ingredients, components, steps, or limitations described herein. Embodiments and aspects described herein may comprise or be combinable with elements, features or components of other embodiments and/or aspects despite not being expressly exemplified in combination, unless an incompatibility is stated.

Where amount ranges are given, these are to be understood as being the total amount of said ingredient in the composition, or where more than one species fall within the scope of the ingredient definition, the total amount of all ingredients fitting that definition, in the composition.

For example, if the composition comprises from 1% to 5% fatty alcohol, then a composition comprising 2% stearyl alcohol and 1% cetyl alcohol and no other fatty alcohol, would fall within this scope.

The amount of each particular ingredient (e.g. a primary intermediate, a coupler, an oxidizing agent, etc) or mixtures thereof described hereinafter can account for up to 100% (or 100%) of the total amount of the ingredient(s) in the first composition and/or the second composition.

The term "substantially free of" as used herein means less than 1%, less than 0.8%, less than 0.5%, less than 0.3%, or less than an immaterial amount of by total weight of the composition.

The term "hair" as used herein means mammalian hair including scalp hair, facial hair and body hair, more preferably hair on the human head and scalp. Hair comprises hair fibers. "Hair shaft" means an individual hair strand and may be used interchangeably with the term "hair." As used herein the term "hair" to be treated may be "living" i.e. on a living body or may be "non-living" i.e. in a wig, hairpiece or other aggregation of non-living keratinous fibers. Mammalian, preferably human hair is preferred. However wool, fur and other keratin containing fibers are suitable substrates for the hair coloring compositions according to the present invention.

By "dye composition", it is meant a composition suitable for changing the color of hair. The dye composition can comprise oxidative dye precursors, direct dyes.

The term "cosmetically acceptable" as used herein means that the compositions, or components described are suitable for use in contact with human keratinous tissue without undue toxicity, incompatibility, instability, allergic response, and the like. All compositions described herein which have the purpose of being directly applied to keratinous tissue are limited to those being cosmetically acceptable.

The term "cosmetically acceptable salt" as used herein refers to conventional base-addition salts that retain the properties of the one or more ionizable linkers of Formula I or the one or more ingredients of formula II of the present invention and are formed from suitable organic or inorganic bases. Sample base-addition salts include those derived from sodium, potassium, ammonium, calcium, magnesium, iron, zinc, zirconium and aluminium hydroxide. Chemical modification of a compound bearing a carboxylic acid function into the corresponding carboxylate salt is a technique well known in the art.

The term "viscosity" as used herein is measured at 25°C using a HAAKE Rotation Viscometer VT 550 with cooling/heating vessel and sensor systems according to DIN 53019 at a shear rate of 12.9 s⁻¹.

The term "separately packaged" as used herein means any form of packaging that prevents a first composition from coming into physical contact, or admixing, with a second composition. "Separately packaged" may mean that the individual first and second compositions are packaged in separate containers, or alternatively in a single container partitioned such that the first and second compositions are not in physical contact.

The term "kit" as used herein means a packaging unit comprising a plurality of components i.e. a kit of parts. An example of a kit is, for example, a first composition and a separately packaged second composition. Another kit may comprise application instructions comprising a method and a composition.

The term "alkyl" as used herein refers to a saturated straight or branched carbon chain. Unless specified otherwise, the alkyl group can have from 1 to 30 carbon atoms, or preferably from 1 to 12 carbon atoms, or more preferably from 1 to 6 carbon atoms. The alkyl groups may also contain one or more heteroatoms within the carbon backbone. Examples include oxygen, nitrogen, sulfur, and combinations thereof. The alkyl group may preferably contain between one and four heteroatoms. The alkyl groups may include straight-chain alkyl or branched-chain alkyl. The term "alkyl" includes both "unsubstituted alkyls" and "substituted alkyls", the latter of which refers to alkyl moieties having one or more substituents replacing a hydrogen on one or more carbons of the hydrocarbon backbone. Such substituents may include, but are not limited to, halogen, hydroxyl, carbonyl (such as a carboxyl, alkoxycarbonyl, formyl, or an acyl), thiocarbonyl (such as a thioester, a thioacetate, or a thioformate), alkoxyl, phosphoryl, phosphate, phosphonate, a phosphinate, amino, amido, amidine, imine, cyano, nitro, azido, sulfhydryl, alkylthio, sulfate, sulfonate, sulfamoyl, sulfonamido, sulfonyl, heterocyclyl, aralkyl, or an aromatic or heteroaromatic moiety.

The term "alkenyl" as used herein is an alkyl containing from 2 to 30 carbon atoms and having one or more double bonds. The alkenyl groups may also contain one or more heteroatoms within the carbon backbone. Examples include oxygen, nitrogen, sulfur, and combinations thereof. The alkenyl group may preferably contain between one and four heteroatoms. The alkenyl groups may include straight-chain alkenyl or branched-chain alkenyl, or cycloalkenyl groups. The term "alkenyl" includes both "unsubstituted alkenyls" and "substituted alkenyls", the latter of which refers to alkenyl moieties having one or more substituents replacing a hydrogen on one or more carbons of the hydrocarbon backbone. Such substituents may be the one as set out hereinbefore in the definition of the term "alkyl".

The term "alkynyl" as used herein is an alkyl containing from 2 to 30 carbon atoms and having one or more triple bonds. The alkynyl groups may also contain one or more heteroatoms within the carbon backbone. Examples include oxygen, nitrogen, sulfur, and combinations thereof. The alkenyl group may preferably contain between one and four heteroatoms. The alkynyl groups may include straight-chain alkynyl or branched-chain alkynyl, or cycloalkynyl groups. The term "alkynyl" includes both "unsubstituted alkynyls" and "substituted alkynyls", the latter of which refers to alkynyl moieties having one or more substituents replacing a hydrogen on one or more carbons of the hydrocarbon backbone. Such substituents may be the one as set out hereinbefore in the definition of the term "alkyl".

The term "cycloalkyl" as used herein represents a cyclic version of "alkyl". The term "cycloalkyl" is also meant to include bicyclic, tricyclic and polycyclic versions thereof. Unless specified otherwise, the cycloalkyl group can have 3 to 12 carbon atoms. By analogy, the term "cycloalkenyl" as used herein represents a cyclic version of "alkenyl". The term "cycloalkynyl" as used herein represents a cyclic version of "alkynyl".

The term "heterocyclyl" as used herein refers to a cyclic radical attached via a ring carbon or nitrogen of a monocyclic or bicyclic ring containing 3-10 ring atoms, or preferably from 5-6 ring atoms, containing carbon and one to four heteroatoms each selected from oxygen, sulfur, and N(Y) wherein Y is absent or is hydrogen, oxygen, (C₁₋₄) alkyl, phenyl or benzyl, and optionally containing one or more double or triple bonds, and optionally substituted with one or more substituents. Examples of heterocyclic ring include, but are not limited to, benzimidazolyl, benzofuranyl, decahydroquinolinyl, 2*H*,6*H-*1,5,2-dithiazinyl, dihydrofuro[2,3-*b*]tetrahydrofuran, imidazolidinyl, imidazolinyl, morpholinyl, octahydroisoquinolinyl, oxazolidinyl, piperazinyl, piperidinyl, piperidonyl, 4-piperidonyl, pyranyl, pyrazolidinyl, pyrazolinyl, pyrrolidinyl, pyrrolinyl, 2H-pyrrolyl, quinuclidinyl and tetrahydrofuranyl.

The term "halogen" as used herein represents fluorine, chlorine, bromine and iodine.

The term "aryl" as used herein refers to an aromatic monocyclic ring containing 6 carbon atoms, an aromatic bicyclic ring system containing 10 carbon atoms or an aromatic tricyclic ring system containing 14 carbon atoms. Examples are phenyl, naphthyl, phenoxathinyl, piperonyl or anthracenyl, preferably phenyl.

The term "heteroaryl" as used herein refers to from three to ten-membered aromatic ring, prefereably a five-or six-membered aromatic ring wherein one or more of the carbon atoms in the ring have been replaced by 1, 2, 3, or 4 (for the five-membered ring) or 1, 2, 3, 4, or 5 (for the six-membered ring) of the same or different heteroatoms, whereby the heteroatoms are selected from the group consisting thereof oxygen, nitrogen, sulfur and mixtures thereof. Examples of the heteroaryl group include groups based on pyrrole, furan, imidazole, pyrazole, oxazole, thiazole, and pyridine. Examples of heteroaryl groups may also include, but are not limited to, benzimidazolyl, benzofuranyl, benzothiofuranyl, benzothiophenyl, benzoxazolyl, benzoxazolinyl, benzthiazolyl, benztriazolyl, benztetrazolyl, benzisoxazolyl, benzisothiazolyl, benzimidazolinyl, carbazolyl, 4a*H*-carbazolyl, chromanyl, chromenyl, cinnolinyl, furanyl, furazanyl, imidazolyl, 1*H*-indazolyl, indolenyl, indolinyl, indolizinyl, indolyl, 3*H*-indolyl, isatinoyl, isobenzofuranyl, isochromanyl, isoindazolyl, isoindolinyl, isoindolyl, isoquinolinyl, isothiazolyl, isoxazolyl, methylenedioxyphenyl, naphthyridinyl, oxadiazolyl, 1,2,3-oxadiazolyl, 1,2,4-oxadiazolyl, 1,2,5-oxadiazolyl, 1,3,4-oxadiazolyl, oxazolyl, oxindolyl, pyrimidinyl, phenanthridinyl, phenanthrolinyl, phenazinyl, phenothiazinyl, phenoxazinyl, phthalazinyl, pteridinyl, purinyl, pyrazinyl, pyrazolyl, pyridazinyl, pyridooxazolyl, pyridinyl, pyrimidinyl, pyrrolyl, quinazolinyl, quinolinyl, 4*H*-quinolizinyl, quinoxalinyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl and thienyl.

The term "acyl" as used herein refers to an alkanoyl group which is usually derived from a carboxylic acid. Therefore, it has the formula RC(O)-, where R represents an alkyl group that is attached to the C(O) group with a single bond.

The term "carboxylic acid" as used herein refers to the group -COOH. Unless specified otherwise the term "carboxylic acid" embraces both the free acid and carboxylate salt.

The term "substituted" as used herein refers to refers to all permissible substituents of the compounds described herein. In the broadest sense, the permissible substituents include acyclic and cyclic, branched and unbranched, carbocyclic and heterocyclic, aromatic and nonaromatic substituents of organic compounds. Illustrative substituents include, but are not limited to, halogens, hydroxyl or alkoxy groups, or any other organic groups containing any number of carbon atoms, preferably C₁₋₁₄ carbon atoms, and optionally include one or more heteroatoms such as oxygen, sulfur, or nitrogen grouping in linear, branched, or cyclic structural formats. Heteroatoms, such as nitrogen, may have hydrogen substituents and/or any permissible substituents of organic compounds described herein that satisfy the valences of the heteroatoms. It is understood that the term "substituted" includes the implicit proviso that such substitution is in accordance with permitted valence of the substituted atom and the substituent, and that the substitution results in a stable compound, *i.e.* a compound that does not spontaneously undergo transformation such as by rearrangement, cyclization, elimination, etc.

The term "polyfunctional molecule" as used herein refers to molecules with more than one functional groups. The functional groups may be the same or different. A functional group can include, but are not limited to alkane, alkene, alkyne, benzene derivative, haloalkane, alcohol, ketone, aldehyde, acyl halide, carbonate, carboxylate, carboxylic acid, ester, alkoxy, ether, heterocyclic, amide, amine, imine, imide, nitrate, nitrile, pyridine, sulfone, sulfoxide. The polyfunctional molecule may preferably contains at least one ionizable functional group capable of forming ionic bonds. The polyfunctional compounds may more preferably contain at least two ionizable groups.

The term "ionizable functional group" as used herein refers to a group capable of forming ionic bonds, like an ammonium group, a carboxylate group or a guanidinium group.

### Method for coloring hair

Hair proteins are the major structural components of the hair. The hair proteins are mostly composed of keratin and keratin associated proteins. The keratin and keratin associated proteins are comprised of specific arrangements of 21 amino acids. The amino acids comprise a large proportion of amino groups and carboxylic acid groups. Also, the keratin and keratin associated proteins contain a large proportion of the amino acid cysteine (circa 17%). Two cysteines can usually form a disulfur bond inside the hair.

The cuticle is the outer protective covering that covers the cortex of each human hair strand and is responsible for the lustre and texture of human hair. The normal cuticle is smooth, allowing light reflection and limiting friction between the hair shafts. It's made up of six to eight layers of flattened overlapping cells and covered by an invisible, water-resistant lipid layer, which acts as a natural conditioner, namely the F-layer. This fatty acid layer (F-layer) is what naturally gives human hair its smooth and silky feel. Chemical processes such as coloring perming and relaxing strip the cuticle of the F-layer, which leads to what is generally referred to as "chemically-damaged" hair.

Due to perhydrolysis upon pre-treatment of the hair (Bleaching process), the F-layer is removed. The epicuticle then possesses on its surface a plurality of sulfonate groups (-SO₃⁻). As a consequence, the interfiber friction increases, enhancing hair breakage.

Also, the disulfur bond between two cysteine amino acids can be broken upon oxidizing treatments such as a bleaching process. The resulting thiol groups of the cysteine can be oxidized into sulfonates during bleaching processes. As a result, the tensile strength of hair decreases, promoting readily hair breakage, hair stiffness, thus an unacceptable loss of hair integrity and healthiness. Furthermore, the swelling is increased, promoting a relatively faster wash-out of dyes.

The present inventor has surprisingly found that when applying sequentially a first composition comprising one or more ionizable linkers of Formula I and a second composition comprising one or more ingredients of Formula II and/or of Formula III, hair integrity and healthiness can be improved. The improvement in hair integrity can be correlated with a reduction in hair stiffness of the hair fibers which is correlated to an increase of flexibility, namely elasticity of the hair fibers. Also, the improvement in hair integrity can be correlated with a reduction in hair breakage of the hair fibers The resulting ionic and hydrogen bonds formed inside the hair fibers can help to increase the flexibility of the hair fibers. The structure of the hair fibers is therefore improved with a reduction of hair breakage and made more resistant to oxidizing agents (bleaching compounds) and/or oxidative dye precursors.

Without wishing to be bound by theory, it is believed that the one or more ionizable linkers of Formula I e.g. *O,O'*-Bis(3-aminopropyl)diethylene glycol can cross-link the carboxylic acid groups of the hair proteins as the hair proteins are made up of amino-acids, by forming hydrogen bonds or ionic bonds. When the F-layer has been removed due to perhydrolysis, the one or more ionizable linkers of the first composition can also interact with the sulfonate groups of the epiticule layer by forming ionic bonds or hydrogen bonds. The one or more ionizable linkers may also cross-link the sulfonate groups of the cysteine amino acids of the hair proteins. Similarly, it is also assumed that the one or more ingredients of Formula II, e.g. malic acid and/or Formula III, e.g. maleic acid can cross-link the amino groups of the hair proteins by forming ionic bonds or hydrogen bonds. The one or more ingredients of Formula II and/or Formula III, may also cross-link the sulfonate groups of the cysteine amino acids of the hair proteins by forming hydrogen bonds. The resulting ionic and hydrogen bonds formed inside the hair fibers can help to increase the flexibility of the hair fibers, which is correlated to a decrease of hair stiffness and also a decrease of hair breakage. The structure of the hair fibers is therefore improved and made more resistant to oxidizing agents and/or oxidative dye precursors.

Human hair fibers are usually dyed with dye compositions containing oxidative dye precursors. Oxidative dye precursors, or oxidation bases, are colorless or slightly colored compounds which, when mixed with oxidizing products at the time of use, are able, by a process of oxidative condensation, to give rise to colored compounds and dyes. It is also known that it is possible to vary the shades obtained with these oxidation bases by combining them with couplers or dye modifiers.

However, oxidative dye precursors are very sensitive when mixed with other active ingredients. Oxidative dye precursors can react with organic compounds to form side products. These side products can have an impact of the final coloration of the hair fibers and alter the final shade. When treating the hair fibers to improve the integrity of hair fibers and coloring the hair fibers at the same time, the present inventor has found that it is possible to combine a dye composition comprising oxidative dye precursors with only the one or more ingredients of Formula II and/or Formula III, and not with the one or more ionizable linkers of Formula I.

Indeed, the one or more ionizable linkers of Formula I as described in more detailed below comprises two ionizable functional groups X and Y; wherein the one or more ionizable functional groups X, Y are independently selected from the group consisting of: -C(O)NR¹R²,-NR¹R², -NR¹S(O)₂R², -NR¹C(O)R². The ionizable functional groups, especially when it is an amino group NR¹R² might interfere with the oxidative dye precursors. Undesirable dyeing side products might be formed. In the contrary, due their acidic nature, the one or more ingredients of Formula II and/or Formula III do not interfere with oxidative dye precursors and do not alter the final color of the hair fibers colored according to the method of the present invention.

Hence, the present invention is related to a method for coloring hair as stated hereinbefore. The method for coloring hair comprises as a step (a), applying to the hair a first composition.

The first composition for treating hair comprises in a cosmetically acceptable carrier, one or more ionizable linkers of Formula I, or cosmetically acceptable salts thereof, or mixtures thereof:

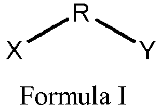

The one or more ionizable linkers is a polyfunctional molecule wherein the R group of the one or more ionizable linkers is an alkyl group, alkenyl group, cycloalkyl group, cycloalkenyl group, aryl group, heterocyloalkyl group, or hetereoaryl group. The alkyl group, alkenyl group, cycloalkyl group, cycloalkenyl group, aryl group, heterocyloalkyl group, or hetereoaryl group is unsubstituted or substituted one or more times by hydrogen, halogen, hydroxy, alkyl, alkenyl, alkynyl, alkoxy, cycloalkyl, cycloalkenyl, cycloalkynyl, aryl, heterocyclyl, heteroaryl, imine, amine, formyl, acyl, carboxylic acid, -C(O)R¹, -C(O)OR¹, (-COO⁻), -CONH₂, -CONHR¹,-C(O)NR¹R², -NR¹R²,-NR¹S(O)₂R², -NR¹C(O)R², -S(O)₂R², -SR¹, -S(O)₂NR¹R², -SOR¹, or-SOOR¹ and mixtures thereof.

The one or more ionizable linkers comprises two ionizable functional groups X and Y; wherein the one or more ionizable functional groups X, Y are independently selected from the group consisting of: -C(O)NR¹R², -NR¹R², -NR¹S(O)₂R², -NR¹C(O)R².

R¹ and R² are each independently selected from the group consisting of a hydrogen, alkyl, alkenyl, alkynyl, cycloalkyl, cycloalkenyl, cycloalkynyl, aryl, heterocyclyl, and heteroaryl group.

Hence, the one or more ionizable linkers of Formula I can possess one or more substituents that is able to form an ionic bond or a hydrogen bond with a group of an amino acid such as a carboxylic acid or an amino group. Also, the one or more ionizable linkers of Formula I can possess one or more substituents may be able to form an ionic bond or a hydrogen bond with another group comprised in a typical damaged hair such as a sulfonate group or a thiolate group.

The one or more ionizable linkers of Formula I may have a length from 2 to 12 carbon atoms, or from 2 to 6 carbon atoms. The one or more ionizable linkers of Formula I can have a size, which can help to cross-link a plurality of carboxylic acids, or sulfonate groups of the amino acids of the hair proteins, preferably by forming ionic bonds or hydrogen bonds.

The one or more ionizable linkers of Formula I may have from 2 to 4 amino substituents. The one or more ionizable linkers of Formula I may preferably have two terminal amino groups.

The one or more ionizable linkers of Formula I may be selected from the group consisting of: 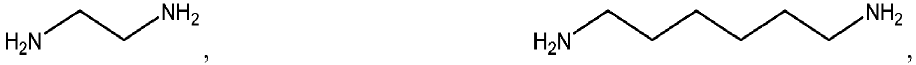 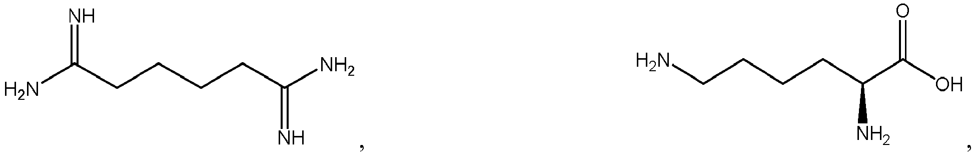 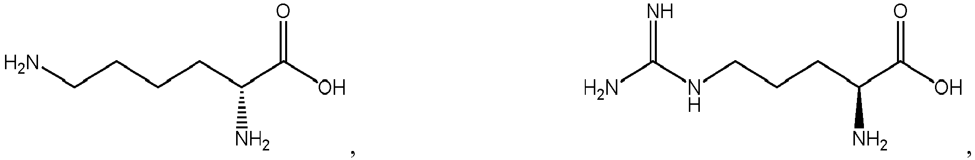 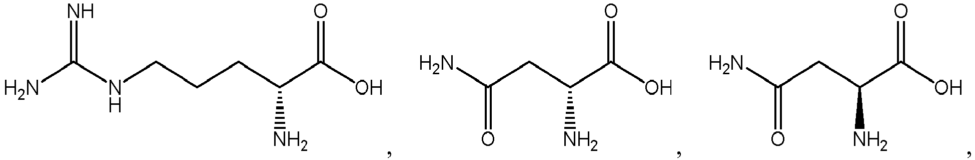 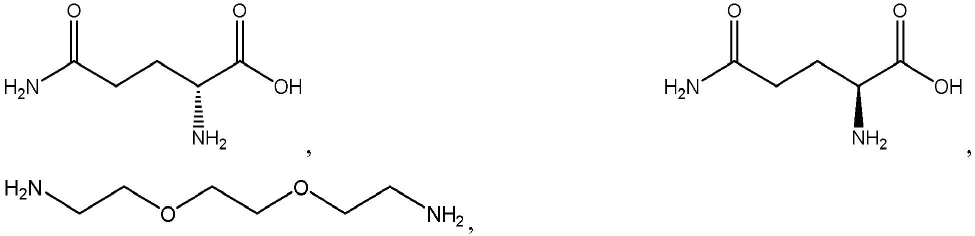 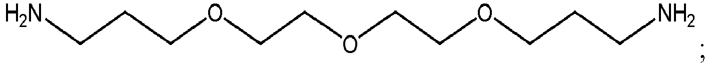 cosmetically acceptable salts thereof, and mixtures thereof.

The one or more ionizable linkers of Formula I may be preferably selected from the group consisting of: 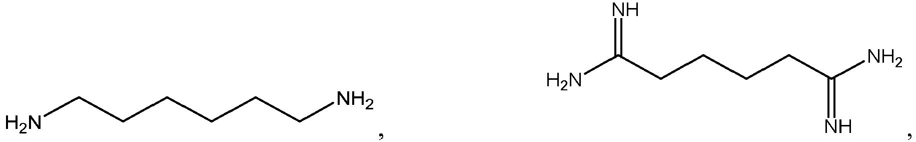 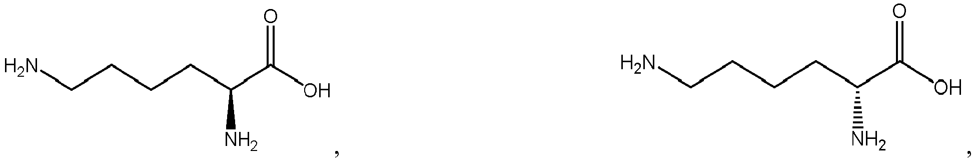 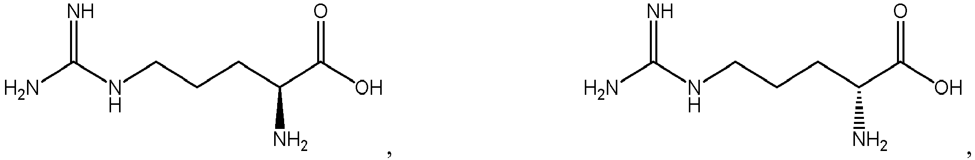 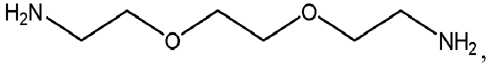 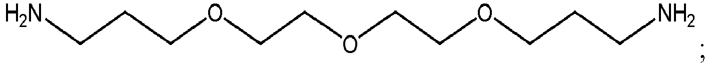 cosmetically acceptable salts thereof, and mixtures thereof.

The one or more ionizable linkers of Formula I may be more preferably selected from the group consisting of: 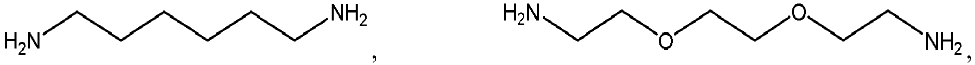 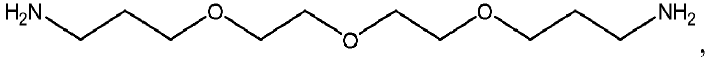 cosmetically acceptable salts thereof, and mixtures thereof.

The first composition may have a pH above 7 to 14, or from 8 to 14, or from 10 to 14, or from 12 to 14. When a first composition has a pH above 7 to 14, the first composition is basic. Hence, especially when the one or more ionizable linkers of Formula I may possess one or more amino groups, preferably one or more terminal amino groups, the one or more ionizable linkers of Formula I can form an ionic bond or a hydrogen bond with either the available carboxylic groups of the amino acids of the hair proteins and/or the sulfonate groups available on the epiticule surface, or the sulfonate groups of the oxidized cysteine amino acids of the hair proteins.

The first composition may comprise from 0.1% to 25%, or from 1% to 20%, or from 2% to 15%, or from 3% to 12% of the one or more ionizable linkers of Formula I by total weight of the first composition.

The method for coloring hair further comprises as a step (b), applying to the hair a second composition comprising in a cosmetically acceptable carrier, one or more ingredients of Formula II, or cosmetically acceptable salts thereof, or mixtures thereof: 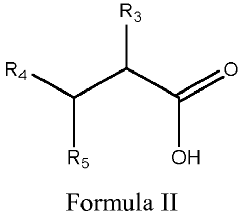
wherein R₃ is independently selected from the group consisting of: -H, -OH, oxo, -H₂N, -C₁₋₆ alkyl groups, -C₁₋₆ alkoxy groups, -C₁₋₆ alkenyl groups;
wherein R₄ is independently selected from the group consisting of: -H, -OH, oxo, -H₂N, -C₁₋₆ alkyl groups, -C₁₋₆ alkoxy groups, -C₁₋₆ alkenyl groups;
wherein R₅ is independently selected from the group consisting of: -H, -C₁₋₆ alkyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid, and ionizable functional groups; and
wherein the ionizable functional group is independently selected from the group consisting of:-COOH, -SO₃H, -PO₃H₂.

The one or more ingredients of Formula II may be preferably selected from the group consisting of acetic acid, glycolic acid, propionic acid, pyruvic acid, lactic acid, glyceric acid, butyric acid, isobutyric acid, valeric acid, isovaleric acid, caproic acid, pivalic acid, malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid, sulfoacetic acid, phosphonoacetic acid; and the cosmetically acceptable salts thereof, and mixtures thereof.

The one or more ingredients of Formula II may be more preferably selected from the group consisting of malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid; and the cosmetically acceptable salts thereof, and mixtures thereof.

Alternatively or in addition to one or more ingredients of Formula II, the method for coloring hair further comprises as a step (b), applying to the hair a second composition comprising in a cosmetically acceptable carrier, one or more ingredients of Formula III, or cosmetically acceptable salts thereof, or mixtures thereof: 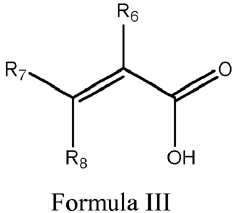
wherein R₆ is independently selected from the group consisting of: -H, -C₁₋₆ alkyl groups, aryl groups, and ionizable functional groups;
wherein R₇ is independently selected from the group consisting of: -H, -C₁₋₆ alkyl groups, aryl groups, and ionizable functional groups;
wherein R₈ is independently selected from the group consisting of: -H, -C₁₋₆ alkyl groups, -C₁₋₆ alkenyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid; and ionizable functional groups; and
wherein the ionizable functional group is independently selected from the group consisting of:-COOH, -CH₂COOH, -SO₃H, -PO₃H₂.

The one or more ingredients of Formula III may be preferably selected from the group consisting of: 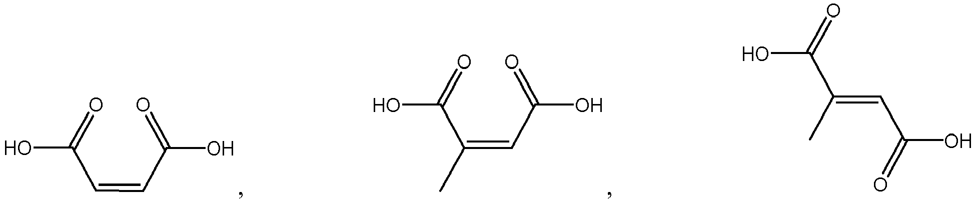 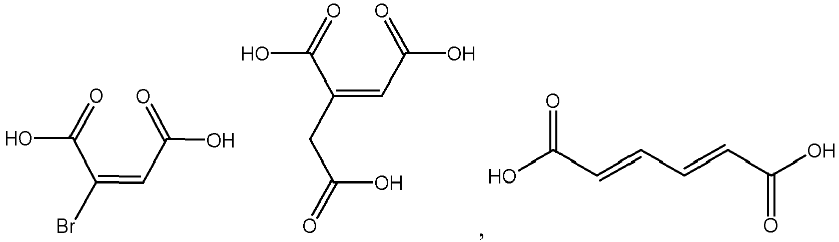 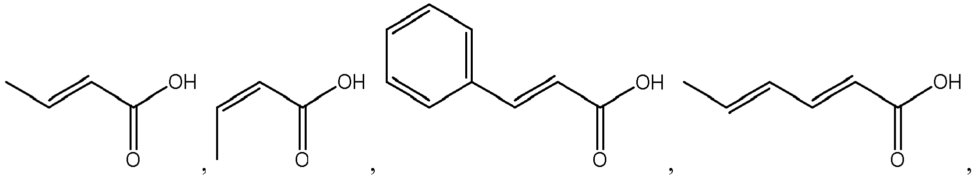 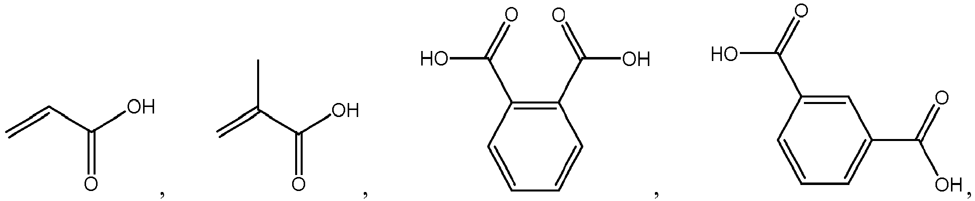 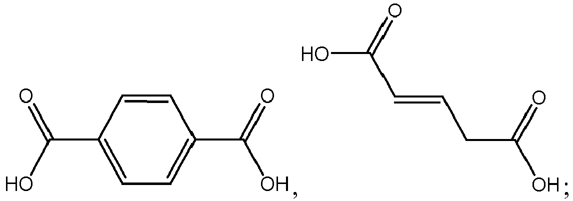 and the cosmetically acceptable salts thereof, and mixtures thereof.

The one or more ingredients of Formula III may be more preferably selected from the group consisting of: 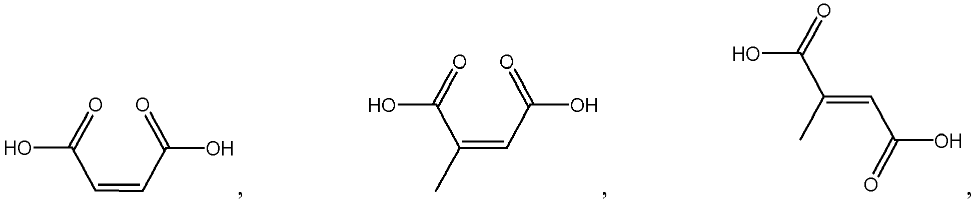 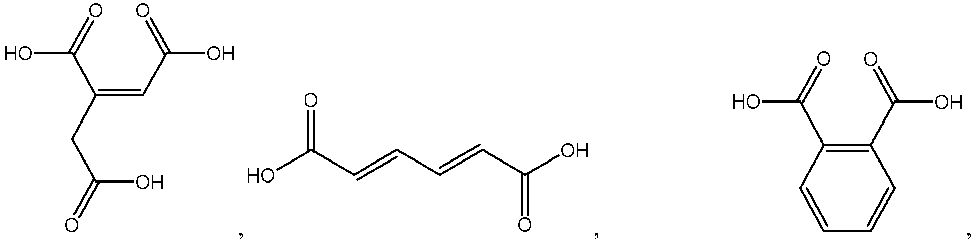 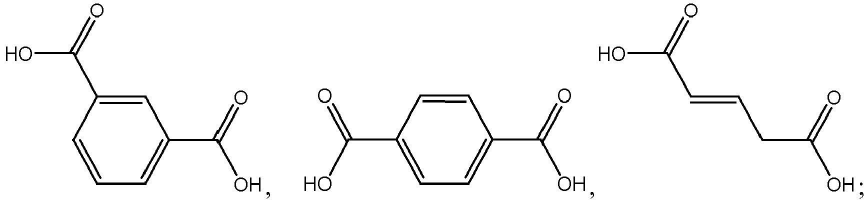 and the cosmetically acceptable salts thereof, and mixtures thereof.

The one or more ingredients of Formula III may be even more preferably selected from the group consisting of: 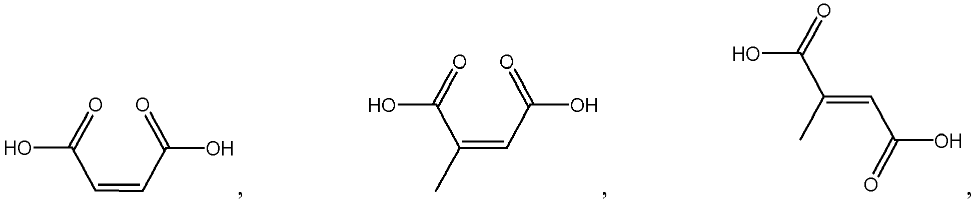 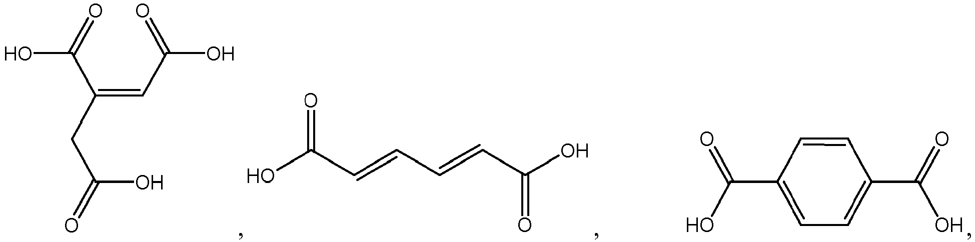 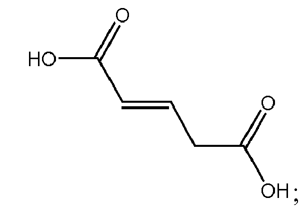 and the cosmetically acceptable salts thereof, and mixtures thereof.

The one or more ingredients of Formula II and/or Formula III possess ionizable functional groups and/or function, e.g. carboxylic acid that are able to also form an ionic bond or a hydrogen bond with a group of an amino acid such as an amino group.

The second composition may have a pH from 0 to 7, or from 0.1 to 5, or from 0.5 to 3, or from 0.5 to 1.5. When a second composition has a pH from 0 to 7, the second composition is acidic. Hence, especially when the one or more ingredients of Formula II and/or Formula III may possess one or more carboxylic groups, preferably one or more terminal carboxylic acid groups, the one or more ingredients of Formula II and/or Formula III can form an ionic bond or a hydrogen bond with the available amino group of the amino acids of the hair proteins, or with the available sulfonate group of the oxidized cysteine amino acids of the hair proteins.

The second composition may comprise from 0.1% to 25%, or from 1% to 20%, or from 2% to 15%, or from 3% to 12% of the one or more ingredients of Formula II, preferably Formula III by total weight of the second composition.

The first composition does not comprise any ingredients of Formula II and/or Formula III of the second composition. The second composition may not comprise any ionizable linkers of Formula I of the first composition. Hence, the one or more ionizable linkers of Formula I of the first composition and the one or more ingredients of Formula II and/or Formula III of the second composition cannot form relatively stable complexes prior to reacting inside the hair. By prior to reacting inside the hair, it is understood by prior to form any ionic bond or other type of bond, e.g. hydrogen bond with the amino acid of the hair proteins.

The first composition does not comprise any oxidative dye precursors. Rather, the second composition further comprises a dye composition and optionally a developer composition wherein the developer composition comprises one or more oxidizing agents; and wherein the dye composition comprises one or more oxidative dye precursors comprising one or more couplers and one or more primary intermediates. The one or more oxidative dye precursors of the dye composition cannot therefore react with the one or more ionizable linkers of Formula I in order to form any indesirable colored side products on the hair fibers. The method of the present invention allows improving the integrity of the hair fibers while dyeing the hair fibers. Also, the method of the present invention allows preventing any formation of side products that could have be obtained with the reaction between the one or more ionizable linkers of Formula I and the oxidative dye precursors.

The one or more ionizable linkers of Formula I may be selected from the group consisting of: 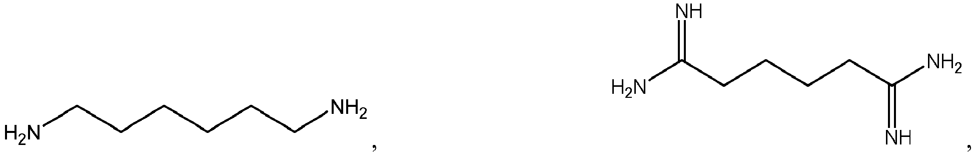 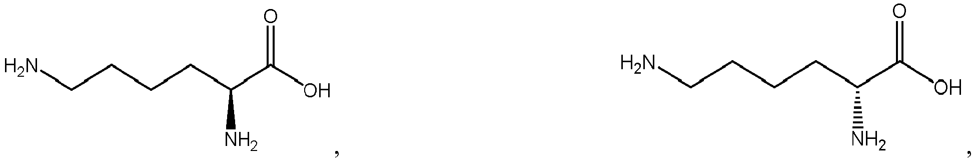 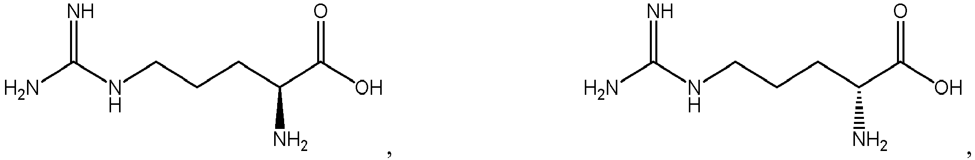 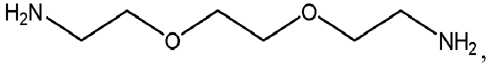 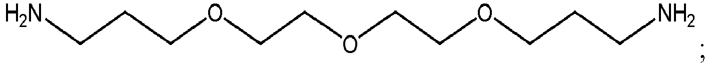 cosmetically acceptable salts thereof, and mixtures thereof; and the one or more ingredients of Formula II may be selected from the group consisting of malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid; and the cosmetically acceptable salts thereof, and mixtures thereof.

The one or more ionizable linkers of Formula I may be selected from the group consisting of: 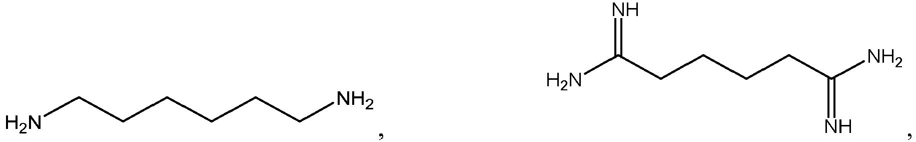 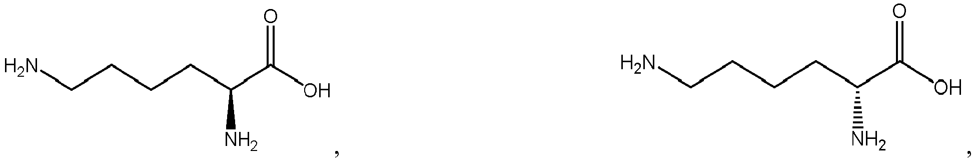 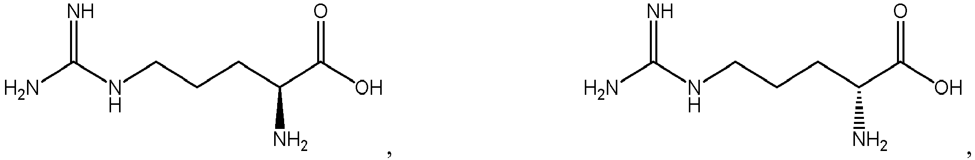 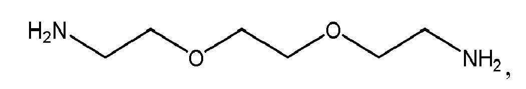 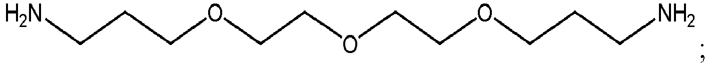 cosmetically acceptable salts thereof, and mixtures thereof; and the one or more ingredients of Formula III may be selected from the group consisting of 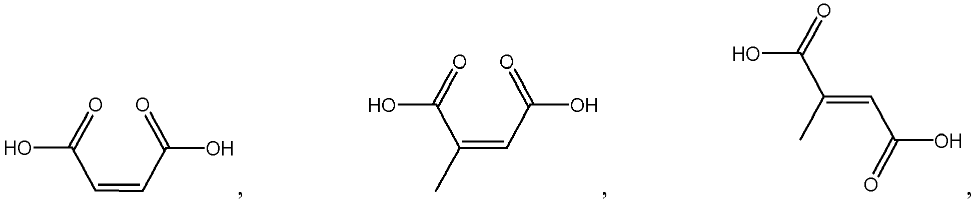 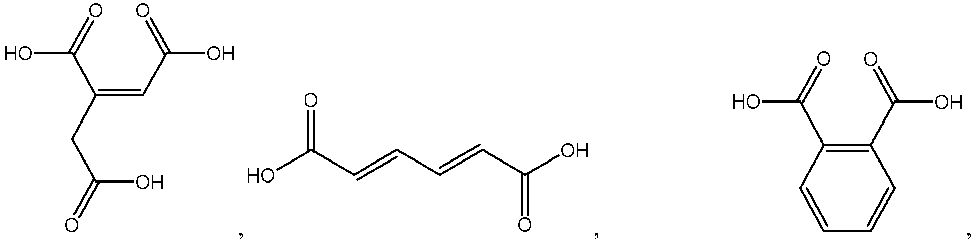 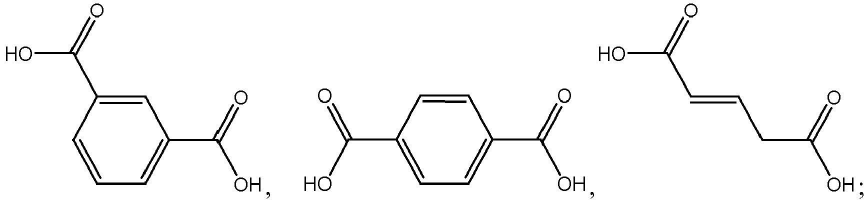 and the cosmetically acceptable salts thereof, and mixtures thereof.

The step (b) of the method may preferably occur prior to the step (a). The step order namely step (b) prior to step (a) is preferred as the one or more ingredients of Formula II and/or Formula III together with the dye composition can be applied on dry hair before the application of the one or more linkers of Formula I. Hence, the dye composition is not in contact with wet hair fibers and wet skin of the head, which can prevent any skin irritation with the components of the dye composition and optionally the developer composition. Alternatively, the step (a) of the method may occur prior to the step (b).

The method may further comprise as a step (c), the step of rinsing, shampooing, conditioning the hair, or a combination thereof. The step (c) may occur subsequent to step (a) and/or step (b).

The step (b) may occur prior to the step (a). In that case, the step (c) may occur subsequent to step (a).

The step (a) may preferably occur prior to the step (b). In that case, the step (c) may occur subsequent to step (a). Hence, the step of rinsing in step (c) can guarantee that no ionizable linkers of Formula I can react with oxidative dye precursors of the dye composition.

### pH

As stated herein before, the first composition may have a pH from 7 to 14, preferably more than 7 to 14, or from 8 to 14, or from 10 to 14, or from 12 to 14. The second composition may have a pH from 0 to 7, or from 0.1 to 5, or from 0.5 to 3, or from 0.5 to 1.5.

The first composition and/or the second composition may comprise a pH modifier and/or buffering agent in an amount that is sufficiently effective to adjust the pH of the first composition and/or the second composition to fall within a range prescribed above. Suitable pH modifiers and/or buffering agents for use herein may include, but are not limited to ammonia, alkanolamines such as monoethanolamine, diethanolamine, triethanolamine, monopropanolamine, dipropanolamine, tripropanolamine, 2-amino-2-methyl-1-propanol, and 2-amino-2-hydroxymethyl-1,3,-propandiol and guanidium salts, alkali metal or ammonium hydroxides and carbonates.

Suitable pH modifiers and/or buffering agents may preferably include sodium hydroxide, sodium silicate, sodium meta silicate and ammonium carbonate, and acidulents such as inorganic and inorganic acids, e.g., phosphoric acid, ascorbic acid, hydrochloric acid, and mixtures thereof. The pH of the first composition may be adjusted with hydrochloric acid, or ascorbic acid. The pH of the second composition may be adjusted with ammonia or monoethanol amine.

### Oxidizing agents

The first composition may comprise one or more oxidizing agents. The developer composition comprises one or more oxidizing agents. Preferred oxidizing agents are water-soluble peroxygen oxidizing agents. The one or more oxidizing agents can be valuable for the initial solubilisation and decolorisation of the melanin (bleaching) and accelerate the oxidation of the oxidative dye precursors (oxidative dyeing) in the hair shaft.

The one or more oxidizing agents may be present in an amount sufficient to bleach melanin pigment in hair and/or cause formation of dye chromophores from oxidative dye precursors. Typically, the first composition and/or the developer may comprise a total amount of oxidizing agents ranging from 0.1% to 20%, or from 0.5% to 12%, or from 1% to 10%, or from 2% to 5%, by total weight of the respective first composition and/or the developer composition.

Suitable water-soluble oxidizing agents may include, but are not limited to: inorganic peroxygen materials capable of yielding hydrogen peroxide in an aqueous solution.

Suitable water-soluble peroxygen oxidizing agents may include, but are not limited to: hydrogen peroxide; inorganic alkali metal peroxides (such as sodium periodate and sodium peroxide); organic peroxides (such as urea peroxide and melamine peroxide); inorganic perhydrate salt bleaching compounds (such as the alkali metal salts of perborates, percarbonates, perphosphates, persilicates, persulphates and the like); and mixtures thereof. Inorganic perhydrate salts may be incorporated for example as monohydrates, tetrahydrates. Alkyl/aryl peroxides and/or peroxidases may also be used. Mixtures of two or more such oxidizing agents can be used if desired. The oxidizing agents may be provided in aqueous solution or as a powder which is dissolved prior to use.

The first composition and/or the developer composition may preferably comprise a water-soluble oxidizing agent which is selected from the group consisting of hydrogen peroxide, percarbonates (which may be used to provide a source of both oxidizing agent and carbonate ions), persulphates, and mixtures thereof. The one or more oxidizing agents of the first composition and/or the developer composition may be sodium percarbonate.

The first composition and/or the developer composition may preferably comprise a water-soluble oxidizing agent comprising a powder which comprises a persulphate salt and a metasilicate salt; and a water-soluble oxidizing agent which is selected from the group consisting of hydrogen peroxide, percarbonates, persulphates, and mixtures thereof

The first composition and/or the developer composition may be substantially free of persulfate.

When the second composition of the present invention is obtained by mixing a developer composition and a dye composition prior to use, the oxidizing agent may be present in the developer composition. The developer composition may be based on any desired formulation chassis, including any commercial product, for example an oil-in-water emulsion. Typical developer compositions comprise 6% or 9% of the H₂O₂ relative to the total weight of the developer composition. A preferred example of a developer composition with respectively 6% and 9% H₂O₂, comprises as INCI ingredients: Water, H₂O₂, Cetearyl Alcohol, Ceteareth-25, Salicylic Acid, Phosphoric Acid, Disodium Phosphate, Etidronic Acid. Another preferred example a developer composition comprises as INCI ingredients: Water, H₂O₂, cetearyl alcohol, lanolin alcohol, sodium lauryl sulfate, parfum, salicylic acid, phosphoric acid, disodium phosphate, linalool, hexyl cinnamal, etidronic acid, tocopherol. Another preferred example a developer composition comprises as INCI ingredients: Water, H₂O₂, cetearyl alchohol, lanolin alcohol, sodium lauryl sulfate, parfum, salicylic acid, phosphoric acid, disodium phosphate, linalool, hexyl cinnamal, etidronic acid, tocopherol.

### Oxidative dye precursors

The second composition further comprises a dye composition comprising oxidative dye precursors comprising one or more couplers (also known as secondary intermediate) and one or more primary intermediates (also known as developer). Various couplers may be used with primary intermediates in order to obtain different shades.

The oxidative dye precursors suitable for use herein, in so far as they are bases, may be used as free bases or in the form of any cosmetically acceptable salts obtained with the corresponding organic or inorganic acids, such as hydrochloric, hydrobromic, citric, acetic, lactic, succinic, tartaric, or sulfuric acids, or, in so far as they have aromatic hydroxyl groups, in the form of any cosmetically acceptable salts obtained with the corresponding bases, such as alkali phenolates.

Oxidative dye precursors are known in the art, and include aromatic diamines, aminophenols, aromatic diols and their derivatives (a representative but not exhaustive list of oxidation dye precursors can be found in Sagarin, "Cosmetic Science and Technology, Interscience, Special Edn. Vol. 2 pages 308 to 310). Suitable oxidative dye precursors are also disclosed in the Canadian Patent Application No. CA 2 576 189 A1 - in particular, from Table 1 dye combinations No. 1 to 2394, which span pages 49 to 238. It is to be understood that the one or more primary intermediates and the one or more couplers (collectively known as oxidative dye precursors) detailed below are only by way of example and are not intended to limit the second composition and other aspects herein described. The one or more primary intermediates and the one or more couplers may be used in the form of any cosmetically acceptable salts, for example sulfate salts.

The one or more primary intermediates of the dye composition may be selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, salts thereof and mixtures thereof.

The one or more primary intermediate of the dye composition may be particularly 1,4-diamino-2-(methoxymethyl)-benzene. 1,4-diamino-2-(methoxymethyl)-benzene has the advantage of an improved sensitisation profile (i.e. reduced risks of scalp skin reaction).

The one or more primary intermediate may be 4,5-diamino-1-hexylpyrazole. 4,5-diamino-1-hexylpyrazole may be used as a sulfate salt.

The one or more primary intermediate may be selected from the group consisting of 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, and mixtures thereof; and the cosmetically acceptable salts thereof such as chlorides, sulfates and hemi-sulfates in particular.

The one or more couplers may be a compound comprising at least one phenyl ring substituted with at least one hydroxyl group. The one or more couplers may be selected from the group consisting of resorcinol, 4-chlororesorcinol, 2-chlororesorcinol, 2-methylresorcinol, 4,6-dichlorobenzene-1,3-diol, 2,4-dimethylbenzene-1,3-diol, m-aminophenol, 4-amino-2-hydroxytoluene, 2-methyl-5-hydroxyethylaminophenol, 3-amino-2,6-dimethylphenol, 3-amino-2,4-dichlorophenol, 5-amino-6-chloro-o-cresol, 5-amino-4-chloro-o-cresol, 6-hydroxybenzomorpholine, 2-amino-5-ethylphenol, 2-amino-5-phenylphenol, 2-amino-5-methylphenol, 2-amino-6-methylphenol, 2-amino-5-ethoxyphenol, 5-methyl-2-(methylamino)phenol, 2,4-diaminophenoxyethanol, 2-amino-4-hydroxyethylaminoanisole, 1,3-bis-(2,4-diaminophenoxy)-propane, 2,2'-(2-methyl-1,3-phenylene)bis(azanediyl)diethanol, benzene-1,3-diamine, 2,2'-(4,6-diamino-1,3-phenylene)bis(oxy)diethanol, 3-(pyrrolidin-1-yl)aniline, 1-(3-(dimethylamino)phenyl)urea, 1-(3-aminophenyl)urea, 1-naphthol, 2-methyl-1-naphthol, 1,5-naphthalenediol, 2,7-naphthalenediol, 1-acetoxy-2-methylnaphthalene, 4-chloro-2-methylnaphthalen-1-ol, 4-methoxy-2-methylnaphthalen-1-ol, 2,6-dihydroxy-3,4-dimethylpyridine, 2,6-dimethoxy-3,5-pyridinediamine, 3-amino-2-methylamino-6-methoxypyridine, 2-amino-3-hydroxypyridine, 2,6-diaminopyridine, pyridine-2,6-diol, 5,6-dihydroxyindole, 6-hydroxyindole, 5,6-dihydroxyindoline, 3-methyl-1-phenyl-1H-pyrazol-5(4H)-one, 1,2,4-trihydroxybenzene, 2-(benzo[d][1,3]dioxol-5-ylamino)ethanol (also known as hydroxyethyl-3,4-methylenedioxyaniline), and mixtures thereof.

The oxidative dye precursors may be particularly selected from the group consisting of 1-naphthol, 2,4-diaminophenoxyethanol, toluene-2,5-diamine sulfate, resorcinol, 4-amino-m-cresol, 2-amino-6-chloro-4-nitrophenol, 2-amino-4-hydroxyethylaminoanisole sulfate, hydroxyethyl-3,4-methylenedioxyaniline HCl, 1-hydroxyethyl 4,5-diamino pyrazole sulfate, 4-amino-2-hydroxytoluene, 2-methylresorcinol, m-aminophenol, 2-methyl-5-hydroxyethylaminophenol, and mixtures thereof.

The oxidative dye precursors may comprise preferably 5-amino-4-chloro-o-cresol and 1,4-diamino-2-(methoxymethyl)-benzene. The oxidative dye precursors may comprise more preferably 2,6-diaminopyridine and 1,4-diamino-2-(methoxymethyl)-benzene. The oxidative dye precursors may comprise even more preferably 2,6-dihydroxyethylaminotoluene and 2-methoxymethyl-1,4-diaminobenzene. The oxidative dye precursors may comprise even more preferably 2-methoxymethyl-1,4-diaminobenzene and p-phenylenediamine and/or toluene-2,5-diamine.

Typically, the dye composition may comprise a total amount of oxidative dye precursors, namely the one or more couplers and the one or more primary intermediates, up to 12%, or from 0.001% to 12%, or from 0.01% to 10%, or from 0.3% to 8%, or from 0.05% to 9%, or from 0.5% to 6% of oxidative dye precursors by total weight of the dye composition.

The first composition and/or the second composition may be substantially free of oxidizing agent.

### Direct dye

The second composition may further comprise one or more direct dyes, advantageously one or more oxidatively stable direct dyes.

The second composition may comprise a total amount from 0.001% to 4%, or from 0.005% to 3%, or from 0.01% to 2% of the one or more direct dyes by total weight of the second composition.

The presence of one or more direct dyes and the proportion thereof can help to provide or enhance coloring/dyeing, particularly with regard to the vibrancy of the color that is desired.

The second composition may be substantially free of any direct dyes. Indeed, sometimes consumers prefer direct dye-free compositions.

The one or more direct dyes may be selected from the group consisting of nitro dyes to provide a blue color, nitro dyes to provide wither a red color or a yellow color, quinone dyes, basic dyes, neutral azo dyes, acid dyes, and mixtures thereof. The one or more direct dyes may be a basic dye. The one or more direct dyes may be a neutral azo dye. The one or more direct dyes may be an acid dye.

The one or more direct dyes may be selected from the group consisting of Acid dyes such as Acid Yellow 1, Acid Orange 3, Acid Black 1, Acid Black 52, Acid Orange 7, Acid Red 33, Acid Yellow 23, Acid Blue 9, Acid Violet 43, Acid Blue 16, Acid Blue 62, Acid Blue 25, Acid Red 4, Basic Dyes such as Basic Brown 17, Basic Red 118, Basic Orange 69, Basic Red 76, Basic Brown 16, Basic Yellow 57, Basic Violet 14, Basic Blue 7, Basic Blue 26, Basic Red 2, Basic Blue 99, Basic Yellow 29, Basic Red 51, Basic Orange 31, Basic Yellow 87, Basic Blue 124, 4-(3-(4-amino-9,10-dioxo-9,10-dihydroanthracen-1-ylamino)propyl)-4-methylmorpholin-4-ium-methylsulfate, (E)-1-(2-(4-(4,5-dimethylthiazol-2-yl)diazenyl)phenyl)(ethyl)amino)ethyl)-3-methyl-1H-imidazol-3-ium chloride, (E)-4-(2-(4-(dimethylamino)phenyl)diazenyl)-1-methyl-1H-imidazol-3-ium-3-yl)butane-1-sulfonate, (E)-4-(4-(2-methyl-2-phenylhydrazono)methyl)pyridinium-1-yl)butane-1-sulfbnate, N,N-dimethyl-3-(4-(methylamino)-9,10-dioxo-4a,9,9a,10-tetrahydroanthracen-1-ylamino)-N-propylpropan-1-aminium bromide, Disperse Dyes such as Disperse Red 17, Disperse Violet 1, Disperse Red 15, Disperse Black 9, Disperse Blue 3, Disperse Blue 23, Disperse Blue 377, Nitro Dyes such as 1-(2-(4-nitrophenylamino)ethyl)urea, 2-(4-methyl-2-nitrophenylamino)ethanol, 4-nitrobenzene-1,2-diamine, 2-nitrobenzene-1,4-diamine, Picramic acid, HC Red No. 13, 2,2'-(2-nitro-1,4-phenylene)bis(azanediyl)diethanol, HC Yellow No. 5, HC Red No. 7, HC Blue No.2, HC Yellow No. 4, HC Yellow No. 2, HC Orange No. 1, HC Red No. 1, 2-(4-amino-2-chloro-5-nitrophenylamino)ethanol, HC Red No. 3, 4-amino-3-nitrophenol, 4-(2-hydroxyethylamino)-3-nitrophenol, 2-amino-3-nitrophenol, 2-(3-(methylamino)-4-nitrophenoxy)ethanol, 3-(3-amino-4-nitrophenyl)propane-1,2-diol, HC Yellow No. 11, HC Violet No. 1, HC Orange No. 2, HC Orange No. 3, HC Yellow No. 9, HC Red No. 10, HC Red No. 11, 2-(2-hydroxyethylamino)-4,6-dinitrophenol, HC Blue No. 12, HC Yellow No. 6, HC Yellow No. 12, HC Blue No. 10, HC Yellow No. 7, HC Yellow No. 10, HC Blue No. 9, 2-chloro-6-(ethylamino)-4-nitrophenol, 6-nitropyridine-2,5-diamine, HC Violet No. 2, 2-amino-6-chloro-4-nitrophenol, 4-(3-hydroxypropylamino)-3-nitrophenol, HC Yellow No. 13, 6-nitro-1,2,3,4-tetrahydroquinoxaline, HC Red No. 14, HC Yellow No. 15, HC Yellow No. 14, N2-methyl-6-nitropyridine-2,5-diamine, N1-allyl-2-nitrobenzene-1,4-diamine, HC Red No. 8, HC Green No. 1, HC Blue No. 14, and Natural dyes such as Annato, Anthocyanin, Beetroot, Carotene, Capsanthin, Lycopene, Chlorophyll, Henna, Indigo, Cochineal, and mixtures thereof.

When the second composition of the invention is obtained by mixing a dye composition and a developer composition, the one or more direct dyes are usually incorporated into the dye composition.

### Other ingredients

The first composition and/or the second composition according to the present invention may comprise, in addition to the ingredients indicated above, further ingredients in order to further enhance the properties of the first composition and/or the second composition, as long as these are not excluded by the claims.

Suitable further ingredients may include, but not limited to: pigments, colored material, solvents, radical scavengers, peroxymonocarbonate ions, surfactants, thickening agents, conditioning agents (such as silicones and cationic polymers), cosmetically acceptable carrier, preservatives, perfume and mixtures thereof.

Suitable further ingredients referred to above, but not specifically described below, are listed in the International Cosmetics Ingredient Dictionary and Handbook, (8th ed.; The Cosmetics, Toiletry, and Fragrance Association). Particularly, vol. 2, sections 3 (Chemical Classes) and 4 (Functions), which are useful in identifying specific adjuvants to achieve a particular purpose or multipurpose. A few of these ingredients are discussed hereinbelow, whose disclosure is of course non-exhaustive.

### Pigment

The second composition may comprise one or more pigments. The one or more pigments of the second composition may be a colored pigment which imparts color effects to the second composition or to the hair.

Alternatively, the one or more pigments of the second composition may be a lustre effect pigment which imparts desirable and aesthetically pleasing lustre effects to the second composition or to the keratin fibers of the hair. The color or lustre effects on the keratin fibers of the hair are preferably temporary. Indeed, the color or lustre effects on the keratin fibers of the hair last until the next hair wash and can be removed again by washing the hair with customary shampoos.

The second composition may be substantially free of pigment. Indeed, having the second composition substantially free of pigment can help to prevent the formation of residues, precipitation and/or rough hair feel.

The second composition may comprise one or more pigments having a D₅₀ particle diameter of from 5 µm to 60 µm measured according to the following test method. Particle diameter is represented by D₅₀, which is the median diameter by volume. D₅₀ is measured with a Malvern Mastersizer 2000, which is a laser diffraction particle sizer and it is measured according to ISO 13320:2009(en) with Hydro 2000G or Hydro 2000S where the dispersant is water or ethanol. Detection range is from 0.02 µm to 2000 µm D₅₀ is expressed as x₅₀ in ISO 13320:2009(en). Laser diffraction measures particle size distributions by measuring an angular variation in intensity of light scattered as a laser beam passes through a dispersed particulate sample analyser and the particle size is reported as a volume equivalent sphere diameter. A discussion of calculating D₅₀ is provided in Barber et al, Pharmaceutical Development and Technology, 3(2), 153-161 (1998).

The second composition may comprise a pigment having a D₅₀ particle diameter from 10 µm to 40 µm. The one or more pigments of the second composition may be present in the second composition in an undissolved form. The second composition may comprise from 0.01% to 25%, or from 0.1% to 20%, or from 1% to 15%, or from 4% to 10% of the one or more pigments by total weight of the second composition.

The one or more pigments of the second composition may be a colorant which is virtually insoluble in the second composition, and may be inorganic or organic. Inorganic-organic mixed pigments may be also possible. The second composition may comprise an inorganic pigment. The advantage of an inorganic pigment is its excellent resistance to light, weather and temperature. The inorganic pigment of the second composition may be of natural origin, and may be, for example, derived from a material selected from the group consisting of chalk, ochre, umber, green earth, burnt sienna, and graphite.

The one or more pigments of the second composition may be a white pigment, such as, for example, titanium dioxide or zinc oxide. Alternatively, the one or more pigments of the second composition may be a black pigment, such as, for example, iron oxide black. Alternatively, the one or more pigments of the second composition may be a colored pigment, such as, for example, ultra-marine or iron oxide red, or a lustre pigment, or a metal effect pigment, or a pearlescent pigment, and/or a fluorescent or phosphorescent pigment.

The one or more pigments of the second composition may be colored or a non-white pigment. The one or more pigments of the second composition may be selected from the group consisting of metal oxides, hydroxides and oxide hydrates, mixed phase pigments, sulfur-containing silicates, metal sulfides, complex metal cyanides, metal sulfates, chromates and molybdates, the metals themselves (bronze pigments), and combinations thereof. The one or more pigments of the second composition may be selected from the group consisting of are titanium dioxide (CI 77891), black iron oxide (CI 77499), yellow iron oxide (CI 77492), red and brown iron oxide (CI 77491), manganese violet (CI 77742), ultramarine (sodium aluminium sulfosilicates, CI 77007, Pigment Blue 29), chromium oxide hydrate (CI 77289), Prussian blue (ferric ferrocyanide, CI 77510), carmine (cochineal), and combinations thereof.

The one or more pigments of the second composition may be a pearlescent and colored pigment based on mica which is coated with a metal oxide or a metal oxychloride, such as titanium dioxide or bismuth oxychloride, and optionally further color-imparting substances, such as iron oxides, Prussian blue, ultramarine, and carmine. The color exhibited by the pigment may be adjusted by varying the layer thickness. Such pigments are sold, for example, under the trade names Rona^{®}, Colorona^{®}, Dichrona^{®}, RonaFlair^{®}, Ronastar^{®}, Xirona^{®} and Timiron^{®} all of which are available from Merck, Darmstadt, Germany. For example, Xirona^{®} is a brand for color travel pigments that display color shifting effects depending on the viewing angle and are based on either natural mica, silica or calcium aluminium borosilicate flakes, coated with varying layers of titanium dioxide.

Pigments from the line KTZ^{®} from Kobo Products, Inc., 3474 So. Clinton Ave., So. Plainfield, USA, may be also useful herein, in particular the Surface Treated KTZ^{®} Pearlescent Pigments from Kobo. Particularly useful are KTZ^{®} FINE WHITE (mica and TiO₂) having a D₅₀ particle diameter from 5 µm to 25 µm and also KTZ^{®} CELESTIAL LUSTER (mica and TiO₂, from 10 µm to 60 µm) as well as KTZ^{®} CLASSIC WHITE (mica and TiO₂, from 10 µm to 60 µm). Another useful pigment may be SynCrystal Sapphire from Eckart Effect Pigments, which is a blue powder comprising platelets of synthetic fluorphlogopite coated with titanium dioxide, ferric ferrocyanide and small amounts of tin oxide. Another useful pigment may also be SYNCRYSTAL Almond also from Eckart, which is a beige powder with a copper reflection color and is composed of platelets of synthetic fluorphlogopite and coated with titanium dioxide and iron oxides. Another useful pigment may be Duocrome^{®} RV 524C from BASF, which provides a two color look via a lustrous red powder with a violet reflection powder due to its composition of mica, titanium dioxide and carmine.

The one or more pigments of the second composition may be an organic pigment. The organic pigment of the second composition may be selected from the group consisting of natural pigments sepia, gamboge, bone charcoal, Cassel brown, indigo, chlorophyll and other plant pigments.

The one or more pigments of the second composition may be a synthetic organic pigment. The synthetic organic pigment of the second composition may be selected from the group consisting of azo pigments, anthraquinoids, indigoids, dioxazine, quinacridone, phthalocyanine, isoindolinone, perylene and perinone, metal complex, alkali blue, diketopyrrolopyrrole pigments, and combinations thereof.

The one or more pigments of the second composition may be selected from the group consisting of iron oxide, titanium dioxide, mica, borosilicate, and combinations thereof. The pigment of the second composition may comprise an iron oxide (Fe₂O₃) pigment. The one or more pigments of the second composition may comprise a combination of mica and titanium dioxide.

### Colored material

The second composition may comprise one or more colored materials. The one or more colored materials of the second composition may be particulate in form. The one or more colored materials of the second composition may be selected from the group consisting of colored fibers, colored beads, colored particles such as nano-particles, colored polymers comprising covalently attached dyes, liquid crystals, particles having diffraction properties, UV absorber and photoprotective substances, pressure- or light-sensitive pigments, and combinations thereof

The second composition may be substantally free of colored material. Indeed, having the second composition substantially free of colored material can help to prevent the formation residues and precipitation.

The one or more colored materials of the second composition may be capable of changing color via a mechanism selected from the group consisting of thermochromism, photochromism, hydrochromism, magnetochromism, electrochromism, piezochromism, chemichromism, mechano-optics. Suitable colored material of the second composition may include 3D Magnetic Pigments, Glow Dust, Fluorescent Pigments, Thermo Dust, Chameleon Pigments and other color changing materials from Solar Color Dust (http://solarcolordust.com/).

The second composition may comprise one or more photoprotective substances. The second composition may comprise from 0.01% to 10%, or from 0.1% to 5%, or from 0.2% to 2% of the one or more photoprotective substances by total weight of the second composition. Useful photoprotective substances of the second composition are specified in European Patent Application EP 1 084 696 A1 from §0036 to §0053. The one or more photoprotective substances of the second composition may be selected from the group consisting of 2-ethylhexyl 4-methoxy-cinnamate, methyl methoxycinnammate, 2-hydroxy-4-methoxybenzophenone-5-sulfonic acid, polyethoxylated p-aminobenzoates, di-butyl-hydroxytoluene (BHT), and mixtures thereof.

The second composition may comprise from 0.01% to 10%, or from 0.05% to 5% of one or more particulate substances by total weight of the second composition. The one or more particulate substances of the second composition may be a substance which is solid at room temperature (23°C) and in the form of a particle. The one or more particulate substances of the second composition may be selected from the group consisting of silica, silicates, aluminates, clay earths, mica, and insoluble salts. The one or more particulate substances of the second composition may be selected from the group consisting of insoluble inorganic metal salts, metal oxides, minerals and insoluble polymer particles. The one or more particulate substances of the second composition may be titanium dioxide.

The one or more particulate substances of the second composition may be present in the second composition in an undissolved, or a stably dispersed form, and, following application to the hair and evaporation of the solvent, can deposit on the hair in a solid form.

The one or more particulate substances of the second composition may be selected from the group consisting of silica (silica gel, silicon dioxide) and metal salts, in particular inorganic metal salts. The particulate substance of the second composition may be silica. The one or more particulate substances of the second composition may be selected from the group consisting of metal salts such as alkali metal or alkaline earth metal halides, e.g. sodium chloride or potassium chloride; alkali metal or alkaline earth metal sulfates, such as sodium sulfate or magnesium sulfate.

### Solvent

The first composition and/or the second composition may further comprise one or more solvents. The one or more solvents may be selected from water, or a mixture of water and at least one organic solvent to dissolve the compounds that would not typically be sufficiently soluble in water.

Suitable organic solvents for the first composition and/or the second composition may include, but are not limited to: from C₂ to C₄ lower alkanols (such as ethanol, propanol, isopropanol); aromatic alcohols (such as benzyl alcohol and phenoxyethanol); polyols and polyol ethers (such as carbitols, 2-butoxyethanol, propylene glycol, propylene glycol monomethyl ether, diethylene glycol monoethyl ether, monomethyl ether, hexylene glycol, glycerol, ethoxy glycol, butoxydiglycol, ethoxydiglycerol, dipropyleneglocol, polygylcerol); propylene carbonate; and mixtures thereof.

The one or more solvents of the first composition and/or the second composition may be selected from the group consisting of water, ethanol, propanol, isopropanol, glycerol, 1,2-propylene glycol, hexylene glycol, ethoxy diglycol, and mixtures thereof.

Typically, the first composition and/or the second composition may comprise water as a main ingredient, particularly in a total amount ranging from at least 50%, alternatively from at least 60%, alternatively from at least 70%, by total weight of the respective first composition and/or the second composition. Typically, when present, the first composition and/or the second composition may comprise a total amount of organic solvents ranging from 1% to 30%, by total weight of the respective first composition and/or the second composition.

### Chelants

The first composition and/or the second composition may further comprise one or more chelants (also known as "chelating agent", "sequestering agent", or "sequestrant") in an amount sufficient to reduce the amount of metals available to interact with formulation components, particularly oxidizing agents, more particularly peroxides. Chelants are well known in the art and a non-exhaustive list thereof can be found in AE Martell & RM Smith, Critical Stability Constants, Vol. 1, Plenum Press, New York & London (1974) and AE Martell & RD Hancock, Metal Complexes in Aqueous Solution, Plenum Press, New York & London (1996).

Typically, the first composition and/or the second composition may comprise a total amount of chelants ranging from at least 0.01%, preferably from 0.01% to 5%, more preferably from 0.25% to 3%, even more preferably from 0.5% to 1%, by total weight of the first composition and/or the second composition.

The one or more chelants may be selected from the group consisting of carboxylic acids (such as aminocarboxylic acids), phosphonic acids (such as aminophosphonic acids), polyphosphoric acids (such as linear polyphosphoric acids), their salts thereof, and mixtures thereof.

By "salts thereof", it is meant - in the context of chelants - all salts comprising the same functional structure as the chelant they are referring to and including alkali metal salts, alkaline earth salts, ammonium salts, substituted ammonium salts, and mixtures thereof; alternatively sodium salts, potassium salts, calcium salts, magnesium salts, ammonium salts, and mixtures thereof; alternatively monoethanolammonium salts, diethanolammonium salts, triethanolammonium salts, and mixtures thereof.

The one or more chelants may be one or more aminocarboxylic acid chelants comprising one or more carboxylic acid moieties (-COOH) and one or more nitrogen atoms. The one or more aminocarboxylic acid chelants may be selected from the group consisting of diethylenetriamine pentaacetic acid (DTPA), ethylenediamine disuccinic acid (EDDS), ethylenediamine-N,N'-diglutaric acid (EDDG), 2-hydroxypropylenediamine-N-N'-disuccinic acid (HPDDS), glycinamide-N,N'-disuccinic acid (GADS), ethylenediamine-N-N'-diglutaric acid (EDDG), 2-hydroxypropylenediamine-N-N'-disuccinic acid (HPDDS), ethylenediaminetetraacetic acid (EDTA), ethylenedicysteic acid (EDC), ethylenediamine-N-N'-bis(ortho-hydroxyphenyl acetic acid) (EDDHA), diaminoalkyldi(sulfosuccinic acids) (DDS), N,N'-bis(2-hydroxybenzyl)ethylenediamine-N,N'-diacetic acid (HBED), their salts thereof, and mixtures thereof.

When the second composition of the invention is obtained by mixing a dye composition and a developer composition, the chelants may be incorporated in the dye composition and/or in the developer composition. A chelant is usually present in the developer composition for stability reason.

### Thickeners and/or rheology modifiers

The first composition and/or the second composition may further comprise one or more thickeners in an amount sufficient to provide the composition with a viscosity so that it can be readily applied to the hair without unduly dripping off the hair and causing mess.

Suitable thickeners include, but are not limited to: associative polymers, polysaccharides, non-associative polycarboxylic polymers, and mixtures thereof.

Typically, the first composition and/or the second composition may comprise a total amount of thickeners ranging from at least 0.1%, preferably at least 0.5%, more preferably at least 1%, by total weight of the first composition and/or the second composition.

The one or more thickeners may be a thickening polymer. The first composition and/or the second composition may comprise from 0.1% to 2% of a thickening polymer by total weight of the first composition and/or the second composition.

The thickening polymer may be selected from the group consisting of associative polymers, crosslinked acrylic acid homopolymers, crosslinked copolymers of (meth)acrylic acid and of (C₁-C₆) alkyl acrylate, polysaccharides and mixtures thereof, The thickening polymer of the composition may also serve as conditioning agents, as described below.

As used herein, the expression "associative polymers" means amphiphilic polymers comprising both hydrophilic units and hydrophobic units, for example, at least one C₈ to C₃₀ fatty chain and at least one hydrophilic unit. Associative polymers are capable of reversibly combining with each other or with other molecules.

The associative thickeners may be selected from the group consisting of nonionic amphiphilic polymers comprising at least one hydrophilic unit and at least one fatty-chain unit; anionic amphiphilic polymers comprising at least one hydrophilic unit and at least one fatty-chain unit; cationic amphiphilic polymers comprising at least one hydrophilic unit and at least one fatty-chain unit; and amphoteric amphiphilic polymers comprising at least one hydrophilic unit and at least one fatty-chain unit, and mixtures thereof.

The nonionic amphiphilic polymers comprising at least one fatty chain and at least one hydrophilic unit may be selected from the group consisting of celluloses modified with groups comprising at least one fatty chain (such as hydroxyethylcelluloses modified with groups comprising at least one fatty chain chosen from alkyl, alkenyl and alkylaryl groups); hydroxypropyl guars modified with groups comprising at least one fatty chain; polyether urethanes comprising at least one fatty chain (such as C₈-C₃₀ alkyl or alkenyl groups); copolymers of vinylpyrrolidone and of fatty-chain hydrophobic monomers; copolymers of C₁-C₆ alkyl acrylates or methacrylates and of amphiphilic monomers comprising at least one fatty chain; copolymers of hydrophilic acrylates or methacrylates and of hydrophobic monomers comprising at least one fatty chain, and mixtures thereof.

The nonionic amphiphilic polymers comprising at least one hydrophilic unit and at least one fatty-chain unit may be selected from the group consisting of those polymers comprising at least one fatty-chain allyl ether unit and at least one hydrophilic unit comprising an ethylenic unsaturated anionic monomeric unit (such as a vinylcarboxylic acid unit, particularly a unit chosen from units derived from acrylic acids, methacrylic acids, and mixtures thereof), wherein the fatty-chain allyl ether unit corresponds to the monomer of formula (I1) below

CH₂=C(R₁)CH₂OBₙR (I1)

in which R₁ is chosen from H and CH₃, B is an ethyleneoxy radical, n is chosen from zero and integers ranging from 1 to 100, R is chosen from hydrocarbon-based radicals chosen from alkyl, alkenyl, arylalkyl, aryl, alkylaryl and cycloalkyl radicals, comprising from 8 to 30 carbon atoms, and, further, for example, from 10 to 24 carbon atoms and even further, for example, from 12 to 18 carbon atoms.

The anionic amphiphilic polymers may be selected from the group consisting of those polymers comprising at least one hydrophilic unit of unsaturated olefinic carboxylic acid type, and at least one hydrophobic unit of the type such as a (C₈-C₃₀) alkyl ester or (C₈-C₃₀) oxyethylenated alkyl ester of an unsaturated carboxylic acid, wherein the hydrophilic unit of unsaturated olefinic carboxylic acid type corresponds to, for example, the monomer of formula (I2) below

CH₂=C(R₁)COOH (I2)

in which R₁ is chosen from H, CH₃, C₂H₅ and CH₂COOH (i.e. acrylic acid, methacrylic, ethacrylic and itaconic acid units); and wherein the hydrophobic unit of the type such as a (C₈-C₃₀) alkyl ester or (C₈-C₃₀) oxyethylenated alkyl ester of an unsaturated carboxylic acid corresponds to, for example, the monomer of formula (I3) below

CH₂=C(R₁)COOBₙR₂ (I3)

in which R₁ is chosen from H, CH₃, C₂H₅ and CH₂COOH (i.e. acrylate, methacrylate , ethacrylate and itaconate units), B is an ethyleneoxy radical, n is chosen from zero and integers ranging from 1 to 100, R₂ is chosen from C₈-C₃₀ alkyl radicals, for example, C₁₂-C₂₂ alkyl radical. Anionic amphiphilic polymers may further be cross-linked. The crosslinking agent can be a monomer comprising a group (I4) below

CH₂=C< (I4)

with at least one other polymerizable group whose unsaturated bonds are not conjugated with respect to one another. Mention may be made, for example, of polyallyl ethers such as polyallylsucrose and polyallyl pentaerythritol.

The cationic amphiphilic polymers may be selected from the group consisting of quaternized cellulose derivatives and polyacrylates comprising amino side groups. The quaternized cellulose derivatives may be, for example, chosen from quaternized celluloses modified with groups comprising at least one fatty chain, such as alkyl, arylalkyl and alkylaryl groups comprising at least 8 carbon atoms, and mixtures thereof, quaternized hydroxyethylcelluloses modified with groups comprising at least one fatty chain, such as alkyl, arylalkyl and alkylaryl groups comprising at least 8 carbon atoms, and mixtures thereof. The alkyl radicals borne by the above quaternized celluloses and hydroxyethylcelluloses, for example, contain from 8 to 30 carbon atoms. The aryl radicals, for example, may be chosen from phenyl, benzyl, naphthyl and anthryl groups.

The amphoteric amphiphilic polymers comprising at least one hydrophilic unit and at least one fatty-chain unit, may be made, for example, of methacrylamidopropyltrimethylammonium chloride/acrylic acid/C8-C30 alkyl methacrylate copolymers, wherein the alkyl radical is, for example, a stearyl radical.

The preferred associative polymers may comprise at least one hydrophilic unit which is unsaturated carboxylic acid or its derivatives, and at least one hydrophobic unit which is a C₈ to C₃₀ alkyl ester or oxyethylenated C₈-C₃₀ alkyl ester of unsaturated carboxylic acid. The unsaturated carboxylic acid may be preferably acrylic acid, methacrylic acid or itaconic acid. Commercially available materials include those sold as Aculyn-22 by Rohm & Haas; Permulen TR1, Carbopol 2020, Carbopol Ultrez-21 by Noveon, Structure 2001/3001 by National Starch.

Other preferred associative polymers may include polyether polyurethane, commercially available as Aculyn-44/-46 by Rohm and Haas. Further preferred associative polymers may include cellulose modified with groups comprising at least one C₈-C₃₀ fatty chain, commercially available under the trade name Natrosol Plus Grade 330 CS by Aqualon.

The non-associative cross-linked polycarboxylic polymers may be selected from the group consisting of cross-linked acrylic acid homopolymers, copolymers of acrylic or (meth)acrylic acid and of C₁-C₆ alkyl acrylate or (meth)acrylate, and mixtures thereof. Commercially available materials include those sold as Carbopol 980/981/954/2984/5984 by Noveon, Synthalen M/Synthalen L/Synthalen K by 3V Sigma, Aculyn-33 by Rohm and Haas.

The polysaccharides may be selected from the group consisting of glucans, modified and unmodified starches (such as those derived, for example, from cereals, for instance wheat, corn or rice, from vegetables, for instance yellow pea, and tubers, for instance potato or cassaya), amylose, amylopectin, glycogen, dextrans, celluloses and derivatives thereof (methylcelluloses, hydroxyalkylcelluloses, ethyl hydroxyethylcelluloses, and carboxymethylcelluloses), mannans, xylans, lignins, arabans, galactans, galacturonans, chitin, chitosans, glucuronoxylans, arabinoxylans, xyloglucans, glucomannans, pectic acids and pectins, alginic acid and alginates, arabinogalactans, carrageenans, agars, glycosaminoglucans, gum arabics, gum tragacanths, ghatti gums, karaya gums, carob gums, galactomannans, such as guar gums, and nonionic derivatives thereof (hydroxypropyl guar) and bio-polysaccharides, such as xanthan gums, gellan gums, welan gums, scleroglucans, succinoglycans, and mixtures thereof.

Suitable polysaccharides are described in "Encyclopedia of Chemical Technology", Kirk-Othmer, Third Edition, 1982, volume 3, pp. 896-900, and volume 15, pp. 439-458, in "Polymers in Nature" by E. A. MacGregor and C. T. Greenwood, published by John Wiley & Sons, Chapter 6, pp. 240-328,1980, and in "Industrial Gums-Polysaccharides and their Derivatives", edited by Roy L. Whistler, Second Edition, published by Academic Press Inc..

A preferred polysaccharide may be a bio-polysaccharide, particularly bio-polysaccharides selected from xanthan gum, gellan gum, welan gum, scleroglucan or succinoglycan; commercially available as Keltrol® T by Kelco and Rheozan® by Rhodia Chimie.

Another preferred polysaccharide may be hydroxypropyl starch derivative, particularly hydroxypropyl starch phosphate, commercially available as Structure XL® by National Starch.

Commercially available salt-tolerant thickeners may be selected from the group consisting of xanthan, guar, hydroxypropyl guar, scleroglucan, methyl cellulose, ethyl cellulose (commercially available as Aquacote), hydroxyethyl cellulose (Natrosol), carboxymethyl cellulose, hydroxypropylmethyl cellulose, microcrystalline cellulose, hydroxybutylmethyl cellulose, hydroxypropyl cellulose (Klucel), hydroxyethyl ethyl cellulose, cetyl hydroxyethyl cellulose (Natrosol Plus 330), N-vinylpyrollidone (Povidone), Acrylates/Ceteth-20 Itaconate Copolymer (Structure 3001), hydroxypropyl starch phosphate (Structure ZEA), polyethoxylated urethanes or polycarbamyl polyglycol ester such as PEG-150/Decyl/SMDI copolymer (Aculyn 44), PEG-150/Stearyl/SMDI copolymer (Aculyn 46), trihydroxystearin (Thixcin), acrylates copolymer (Aculyn 33) or hydrophobically modified acrylate copolymers (such as Acrylates / Steareth-20 Methacrylate Copolymer as Aculyn 22), acrylates/steareth-20 methacrylate crosspolymer (Aculyn 88), acrylates/vinyl neodecanoate crosspolymer (Aculyn 38), acrylates/beheneth-25 methacrylate copolymer (Aculyn 28), acrylates/C10-30 alkyl acrylate crosspolymer (Carbopol ETD 2020), non-ionic amphophilic polymers comprising at least one fatty chain and at least one hydrophilic unit selected from polyether urethanes comprising at least one fatty chain, blends of Ceteth - 10 phosphate, Di-cetyl phosphate and Cetearyl alcohol (available as Crodafos CES), and mixtures thereof.

### Surfactants

The first composition and/or the second composition may comprise one or more surfactants. A surfactant can help to provide an emulsion. The first composition and/or the second composition may be in the form of an emulsion.

The first composition and/or the second composition may be in the form of a cream or gel. The first composition and/or the second composition may have a lamellar structure and/or may have a gel network. The first composition and/or the second composition n may comprise micelles comprising a hydrophobic phase (see the description of the hydrophobic phase more below).

The first composition and/or the second composition may comprise from 0.001% to 10%, preferably from 0.1% to 8%, more preferably from 0.5% to 5%, even more preferably from 0.4% to 2%, or even much more preferably from 0.8% to 1.5% of the one or more surfactants by total weight of the first composition and/or the second composition.

The first composition and/or the second composition may comprise one or more surfactants which are selected from the group consisting of anionic surfactants, non-ionic surfactants, amphoteric surfactants, zwitterionic surfactants, cationic surfactants, and mixtures thereof. The one or more surfactants can be useful for stabilising a hydrophobic phase in the first composition and/or the second composition, e.g. for stabilising the gel network and/or lamellar structure.

The first composition and/or the second composition may comprise an anionic surfactant. The anionic surfactants may be selected from the group consisting of salts (such as alkaline salts, for example, sodium salts, ammonium salts, amine salts, amino alcohol salts and magnesium salts) of the following compounds: alkyl sulphates, alkyl ether sulphates, alkylamido ether sulphates, alkylarylpolyether sulphates, monoglyceride sulphates; alkyl sulphonates, alkyl phosphates, alkylamide sulphonates, alkylaryl sulphonates, α-olefin sulphonates, paraffin sulphonates; alkyl sulphosuccinates, alkyl ether sulphosuccinates, alkylamide sulphosuccinates; alkyl sulphosuccinamates; alkyl sulphoacetates; alkyl ether phosphates; acyl sarcosinates; acyl isethionates; N-acyltaurates; and mixtures thereof. The alkyl or acyl radical of all of these various compounds, for example, comprises from 8 to 24 carbon atoms, and the aryl radical, for example, is chosen from phenyl and benzyl groups. Among the anionic surfactants, which can also be used, mention may also be made of fatty acid salts such as the salts of oleic, ricinoleic, palmitic and stearic acids, coconut oil acid or hydrogenated coconut oil acid; acyl lactylates in which the acyl radical comprises from 8 to 20 carbon atoms. Weakly anionic surfactants can also be used, such as alkyl-D-galactosiduronic acids and their salts, as well as polyoxyalkylenated (C₆-C₂₄) alkyl ether carboxylic acids, polyoxyalkylenated (C₆-C₂₄) alkylaryl ether carboxylic acids, polyoxyalkylenated (C₆-C₂₄) alkylamido ether carboxylic acids and their salts, for example, those comprising from 2 to 50 ethylene oxide groups, and mixtures thereof Anionic derivatives of polysaccharides, for example carboxyalkyl ether of alkyl polyglucosides, can be also used.

Suitable anionic surfactant(s) may comprise at least one anionic functional groups at their head selected from sulfate, sulfonate, phosphate and carboxylates.

Suitable alkyl sulfates include ammonium lauryl sulfate, sodium lauryl sulfate (sodium dodecyl sulfate, SLS, or SDS), and alkyl-ether sulfates, such as sodium laureth sulfate (sodium lauryl ether sulfate or SLES), and sodium myreth sulfate.

Further suitable anionic surfactants may include Docusate (dioctyl sodium sulfosuccinate), alkyl-aryl ether phosphate, alkyl ether phosphate, alkyl carboxylate, such as sodium stearate, sodium lauroyl sarcosinate, ammonium laureth sulfate, disodium lauryl sulfosuccinate, and sodium lauryl sulphoacetate.

Preferred anionic surfactants may be selected from the group consisting of sodium laurylethersulfate, sodium laurethethersulfate, sodium dodecyl sulfate, ammonium laurethethersulfat, ammonium dodecyl sulfate, alkylbenzenesulfonate, and combinations thereof.

The one or more surfactants of the composition may be a non-ionic surfactant. The non-ionic surfactant may be selected from the group consisting of lanolin alcohol, and polyoxyethylene ethers of fatty alcohols, and mixtures thereof. The non-ionic surfactant may be preferably ceteareth-n, wherein n is from 2 to 100, or from 10 to 30. When the one or more surfactants of the composition are non-ionic, precipitation of others ingredients of the composition can be prevented. Suitable nonionic surfactants are compounds that are well known (see, for example, in this respect "Handbook of Surfactants" by M. R. Porter, published by Blackie & Son (Glasgow and London), 1991, pp. 116-178).

The the first composition and/or the second composition may comprise from 0.001% to 5%, preferably from 0.01% to 3%, more preferably from 0.01% to 1%, even more preferably from 0.05% to 1%, even much more preferably from 0.1% to 0.5%, or from 0.1% to 0.3% of a non-ionic surfactant by total weight of the first composition and/or the second composition The non-ionic surfactant of the composition may be selected from the group consisting of lanolin alcohol, and polyoxyethylene ethers of fatty alcohols, and mixtures thereof.

### Conditioning agent

The first composition and/or the second composition may comprise one or more conditioning agents. The one or more conditioning agents of the first composition and/or the second composition may be selected from the group consisting of silicone materials, amino silicones, fatty alcohols, polymeric resins, polyol carboxylic acid esters, cationic polymers, cationic surfactants, insoluble oils and oil derived materials and mixtures thereof The one or more conditioning agents of the first composition and/or the second composition may be selected from the group consisting of mineral oils, glycerine, sorbitol and mixtures thereof

The first composition and/or the second composition may comprise from 0.05% to 20%, or from 0.1% to 15%, or from 0.2% to 10%, or from 0.2% to 2%, or from 0.5% to 2% of the one or more conditioning agents by total weight of the respective first composition and/or the second composition. The one or more conditioning agents may be included in a separate pre- and/or post-treatment composition.

Suitable conditioning agents may include, but are not limited to: silicones, aminosilicones, fatty alcohols, polymeric resins, polyol carboxylic acid esters, cationic polymers, cationic surfactants, insoluble oils and oil derived materials and mixtures thereof. Additional conditioning agents may include mineral oils and other oils such as glycerin and sorbitol.

Particularly useful conditioning agents for the first composition and/or the second composition may be cationic polymers and/or silicones. Cationic polymers may be chosen from those comprising units of at least one amine group chosen from primary, secondary, tertiary and quaternary amine groups that may either form part of the main polymer chain, or be borne by a side substituent that is directly attached to the main polymer chain.

The one or more conditioning agents of the first composition and/or the second composition may be a silicone. The silicone of the first composition and/or the second composition may be selected from the group consisting of polyalkylsilioxane oils, linear polydiemthylsiloxane oils containing trimethylsilyl or hydroxydimethylsiloxane endgroups, polymethylphenylsiloxane polydimethylphenylsiloxane, polydimethyldiphenylsiloxane oils, silicone resins, organofunctional siloxanes having in their general structure one or a number of organofunctional group(s), the same or different, attached directly to the siloxane chain, and mixtures thereof. Said organofunctional group(s) may be selected from: polyethyleneoxy and/or polypropyleneoxy groups, (per)fluorinated groups, thiol groups, substituted or unsubstituted amino groups, carboxylate groups, hydroxylated groups, alkoxylated groups, quaternium ammonium groups, amphoteric, betain groups and mixtures thereof. The silicone of the first composition and/or the second composition may be either used as a neat fluid or in the form of an pre-formed emulsion.

### Cosmetically acceptable carrier

The first composition and/or the second composition comprises a cosmetically acceptable carrier. The cosmetically acceptable carrier of the first composition and/or the second composition may be an aqueous carrier. The first composition and/or the second composition may comprise water. Water can provide a hydrophilic phase, which the hydrophilic portions of any other ingredients comprised in the first composition and/or the second composition can interact with water. Water can also provide a fluid phase meaning that the first composition and/or the second composition can be in liquid form and therefore easily mixed with other fluid compositions such as an oxidizing composition. The first composition and/or the second composition may comprise from 50% to 85% water, or from 65% to 75% of water by total weight of the respective first composition and/or the second composition.

The cosmetically acceptable carrier may be any carrier suitable for formulating the one or more ionizable linkers of Formula I or the one or more ingredients of Formula II into the respective first composition and/or the second composition being suitable for application onto hair. The cosmetically acceptable carrier may be selected from either an aqueous medium or an aqueous-alcoholic medium. When the cosmetically acceptable carrier is an aqueous-alcoholic carrier, the cosmetically acceptable carrier may comprise water and an alcohol. An alcohol can advantageously influence the viscosity of a relatively wide spectrum of ingredients of the first composition and/or the second composition. The alcohol of the first composition and/or the second composition may be selected from the group consisting of: ethanol, isopropanol, propanol, and mixtures thereof.

When the cosmetically acceptable carrier is an aqueous carrier, the aqueous carrier may consist essentially of water and may be substantially free of alcohol. The first composition and/or the second composition may comprise a safe and effective amount of cosmetically acceptable carrier which is water. The first composition and/or the second composition may comprise from 0.1% to 99%, or from 1% to 98%, or from 10% to 97%, or from 30% to 95% of water by total weight of the respective first composition and/or the second composition.

The first composition and/or the second composition may be substantially free of alcohol, such as volatile alcohols (e.g. ethanol, isopropanol, propanol). When the first composition and/or the second composition is substantially free of alcohol, the first composition and/or the second composition can have advantageously a reduced odour. Flammability issues can also be prevented.

The cosmetically acceptable carrier of the first composition and/or the second composition may be an oily compound. The oily compound may be selected from the group consisting of cyclic silicones and volatile hydrocarbons. Cyclic silicones can be available from Dow Corning. The cyclic silicone may have from at least 3 silicone atoms or from at least 5 silicone atoms but no more than 7 silicone atoms or no more than 6 silicone atoms. The cyclic silicone may conform to the formula: 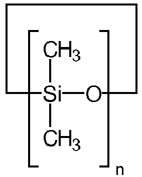 wherein n is from 3 or from 5 but no more than 7 or no more than 6. The cyclic silicone may have a kinematic viscosity of less than 10 cSt at 23°C. A Suitable cyclic silicone for use herein may include Cyclomethicone D5 (commercially available from G.E. Silicones). Alternatively, the first composition and/or the second composition may be silicone-free.

Volatile hydrocarbons e.g. Isopar can be obtained from ExxonMobil Petroleum and Chemical. The oily compound may be a mineral oil. Trade names for suitable mineral oils include Benol, Blandol, Hydrobrite, Kaydol (Sonneborn LLC Refined Products), Chevron Superla White Oil (Chevron Products Company), Drakeol, Parol (Calumet Penreco LLC), Peneteck (Calumet Penreco LLC), Marcol, and Primol 352 (ExxonMobil Petroleum and Chemical).

### Hydrophobic phase

The first composition and/or the second composition may comprise a hydrophobic phase. The hydrophobic phase of the first composition and/or the second composition may be selected from the group consisting of fatty alcohols, fatty acids, and mixtures thereof. The fatty alcohols and/or fatty acids may comprise from 10 to 30, or from 12 to 20, or from 16 to 18 carbon atoms. The hydrophobic phase of the first composition and/or the second composition may comprise two different fatty alcohols. The hydrophobic phase of the first composition and/or the second composition may comprise two different fatty alcohols, both comprising from 10 to 14 carbons.

### Preservative

The first composition and/or the second composition may comprise at least one preservative and/or a mixture of preservatives. The first composition and/or the second composition may comprise from 0.01% to 1% preservative, or from 0.1% to 0.5% preservative by total weight of the respective first composition and/or the second composition. The preservative of the first composition and/or the second composition may be selected from the group consisting of benzyl alcohol, phenoxyethanol, 1,3-bis(hydroxymethyl)-5,5-dimethylimidazolidine-2,4-dione, and mixtures thereof. The first composition and/or the second composition may comprise at least one preservative; and wherein the preservative may be selected from the group consisting of benzyl alcohol, phenoxyethanol, and mixtures thereof; or wherein the preservative may be a mixture of benzyl alcohol and phenoxyethanol. The first composition and/or the second composition may be substantially free of benzoate compounds. Indeed, having benzoate compounds can help to prevent instability and/or precipitation of the first composition and/or the second composition. The first composition and/or the second composition may be substantially free of parabens.

### Perfume

The first composition and/or the second composition may comprise a perfume. The first composition and/or the second composition may comprise from 0.001% to 2% of a perfume by total weight of the respective first composition and/or the second composition. Perfume can provide an enhanced user experience by making the composition smell pleasant and/or invoke emotions tailored to the visual effects on the fibers, such as relaxing or exciting smells.

Alternatively, the first composition and/or the second composition may be substantially free of perfume and/or fragrance. Some consumers prefer perfume-free compositions.

The perfume of the first composition and/or the second composition may be an animal fragrance or a plant fragrance. The animal fragrance may be selected from the group consisting of musk oil, civet, castoreum, ambergris, and mixtures thereof.

The plant fragrance may be selected from the group consisting of nutmeg extract, cardomon extract, ginger extract, cinnamon extract, patchouli oil, geranium oil, orange oil, mandarin oil, orange flower extract, cedarwood, vetyver, lavandin, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract, and mixtures thereof.

The perfume of the first composition and/or the second composition may be selected from the group consisting of acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, p-methylacetophenone, methyl anthranilate, methyl cedrylone, methyl dihydrojasmonate, methyl eugenol, methyl ionone, methyl-α-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox, and mixtures thereof.

### Viscosity

The first composition and/or the second composition may have a kinematic viscosity of from 0.5 cSt to 1500 cSt, measured at 23°C according to the following method. "Viscosity" can mean dynamic viscosity (measured in mPa·s) or kinematic viscosity (measured in centistokes, cSt) of a liquid at 23°C and ambient conditions. Dynamic viscosity may be measured using a rotational viscometer, such as a Brookfield Dial Reading Viscometer Model 1-2 RVT available from Brookfield Engineering Laboratories (USA) or other substitutable model as known in the art. Typical Brookfield spindles which may be used include, without limitation, RV-7 at a spindle speed of 20 rpm, recognizing that the exact spindle may be selected as needed by one skilled in the art. Kinematic viscosity may be determined by dividing dynamic viscosity by the density of the liquid (at 23°C and ambient conditions), as known in the art.

The viscosity of the first composition and/or the second composition may be useful in view of enabling the first composition and/or the second composition to be readily applied to the hair fibers - e.g. spread evenly onto the hair. Viscosity can be influenced by the level of cosmetically acceptable carrier in the first composition and/or the second composition and the level of the thickening agent.

The first composition and/or the second composition may have a kinematic viscosity of from 1 cSt to 1000 cSt. The first composition and/or the second composition may have a kinematic viscosity of from 1.5 cSt to 500 cSt, or from 2 cSt to 350 cSt, or from 2.5 cSt to 200 cSt, or from 3 cSt to 150 cSt, measured at 23°C. 1 centistoke (cSt) is equal to 1 x 10⁻⁶ m²/s).

The first composition and/or the second composition may have a dynamic viscosity of from 1 mPa·s to 5000 mPa·s. The first composition and/or the second composition may have a viscosity of from 2 mPa·s to 400 mPa·s, or from 3 mPa·s to 100 mPa·s. Alternatively, the first composition and/or the second composition may have a dynamic viscosity of from 30 mPa·s to 250 mPa·s, or from 100 mPa·s to 200 mPa·s.

This viscosity range of the first composition and/or the second composition may be useful in view of helping to prevent the first composition and/or the second composition from dripping. When the viscosity is too high, the first composition and/or the second composition may not be readily mixed, e.g. with the cosmetically acceptable carrier, where present.

### Volatility

The first composition and/or the second composition may be substantially free of compounds having a vapor pressure below 0.01 mmHg, or below 0.001 mm Hg, measured at 23°C and 1 atm. Having the first composition and/or the second composition having a relatively low volatility can help to reduce the odour of the first composition and/or the second composition and also can help to provide a relatively safer safety profile.

### Theology

The first composition and/or the second composition may further comprise a hydrophobic phase, a hydrophilic phase, one or more surfactants, and one or more thickening polymers capable of interacting with the hydrophobic phase and the hydrophilic phase, wherein the composition has a storage modulus of at least 3000 Pa, or at least 3300 Pa, or at least 3500 Pa, or at least 4000 Pa, or at least 4500 Pa, or at least 5000 Pa, measured by frequency sweep at an angular frequency of 0.6 rad/s at 23°C, and wherein the one or more thickening polymers are an associative thickening polymer and comprise hydrophobic moieties and hydrophilic moieties. The storage modulus may be not more than 10 kPa, or 9 kPa, or 8 kPa, or 7 kPa, or 6kPa, measured by frequency sweep at an angular frequency of 0.6 rad/s at 23°C. The hydrophilic moieties of the associative thickening polymer may comprise urethane units.

### Kit

A second aspect of the present invention is related to a kit for treating hair comprising:
(a) a first composition comprising in a cosmetically acceptable carrier, one or more ionizable linkers of Formula I as defined hereinbefore;
(b) a second composition comprising in a cosmetically acceptable carrier, one or more ingredients of Formula II and/or Formula III as defined hereinbefore;
(c) a dye composition comprising one or more oxidative dye precursors as defined hereinbefore;
(d) optionally, a developer composition comprising one or more oxidizing agents as defined hereinbefore;
(e) an instructional material for preparation, for use, or both preparation and use, of the first composition, the second composition, the dye composition and optionally the developer composition.

The first composition does not comprise any ingredients of Formula II and/or Formula III of the second composition; and any oxidative dye precursors. The second composition does not comprise any ionizable linkers of Formula I. The first composition, the second composition, the dye composition and optionally the developer composition are separately packaged in different containers or packaged in a same container in different compartments.

The dye composition may be mixed with the second composition, optionally with the developer composition, especially prior to use.

The kit may further comprise a conditioning composition comprising one or more conditioning agents. Conditioning agents have already been described above.

The kit may further comprise a thickening composition. Such thickening compositions are currently on the market as under the brand "Color.id" from Wella Professionals. The thickening composition of the kit may comprise one or more thickening polymers capable of interacting with the hydrophobic phase and the hydrophilic phase.

The kit may further comprise an oxidative composition comprising a water-soluble oxidizing agent preferably comprising a powder which comprises a persulphate salt and a metasilicate salt; and a water-soluble oxidizing agent which is selected from the group consisting of hydrogen peroxide, percarbonates, persulphates, and mixtures thereof.

The kit may further comprise a mixing receptacle and/or a mixing means. The mixing receptacle of the kit may be a bowl. The mixing means of the kit may be a spatula.

The kit comprises an instructional material for preparation, for use, or both preparation and use, of the first composition, the second composition, the dye composition and optionally the developer composition. The instructional material can contain instructions how to use the first and the second compositions, in which order, how to mix the second composition with the dye or the developer compositions or both. Also, the instructional material can contain further instructions how to use and apply the different compositions of the kit.

The first composition and the second composition may be packaged in separate sealed containers. The first composition may be packaged in a flexible tube packaging composed of metal, plastics or a combination thereof. The second composition may be packaged in a squeezable container. This can be applied to the dye composition and the developer composition. The squeezable container may have at least 50% headspace. The squeezable container may have a headspace being at least the volume of the first composition. The first composition may be packaged in a plastic container according to claim 1 of European Patent Application EP 2 801 281 A1, wherein the plastic container has two symmetrical collapsible side panels and a non-collapsible squeezable back panel; wherein the ratio of the average thicknesses between front and/or back panels and the side panels is at least 2:1 (EP 2 801 281 A1 paragraphs [0025] to [0044] as well as the Figures). The plastic container has the advantage that it is resistant to random, uncontrolled deformation under a substantial pressure differential between the environment and inside the container, yet having an affordable cost of manufacture and/or being appealing to the consumer.

The method may be carried out from sequentially applied to the hair, the first composition as stated hereinbefore and the second composition as stated hereinbefore.

In the kit, the one or more ionizable linkers may be selected from the group consisting of: 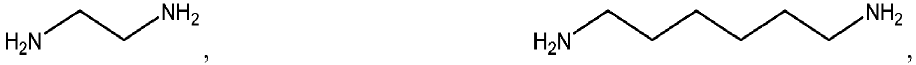 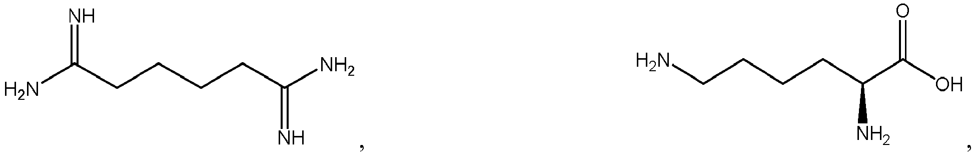 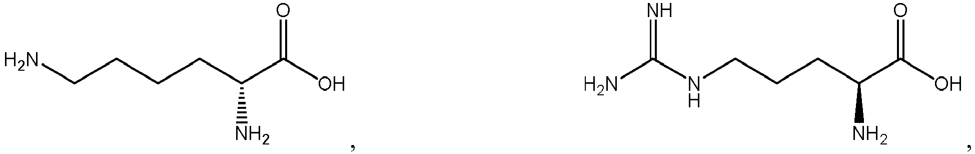 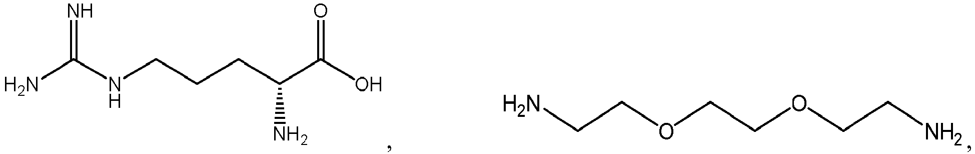 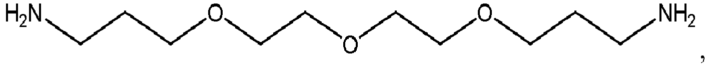 cosmetically acceptable salts thereof, and mixtures thereof; and the one or more ingredients of Formula II may be selected from the group consisting of: acetic acid, glycolic acid, propionic acid, pyruvic acid, lactic acid, glyceric acid, butyric acid, isobutyric acid, valeric acid, isovaleric acid, caproic acid, pivalic acid, malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid, sulfoacetic acid, phosphonoacetic acid; and the cosmetically acceptable salts thereof, and mixtures thereof.

In the kit, the one or more ionizable linkers may be selected from the group consisting of: 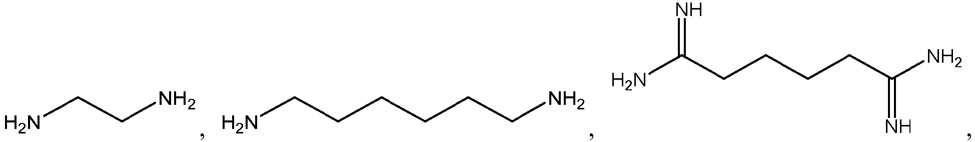 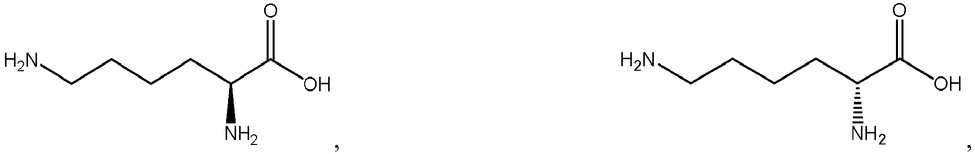 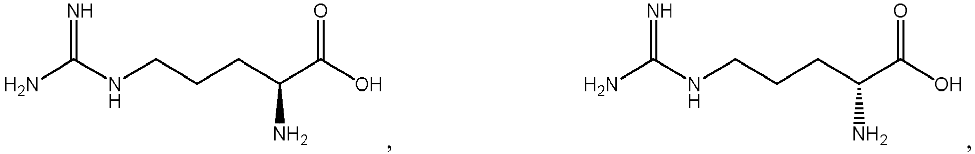 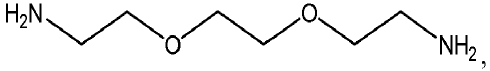 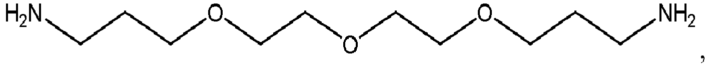 cosmetically acceptable salts thereof, and mixtures thereof; and the one or more ingredients of Formula III may be selected from the group consisting of: 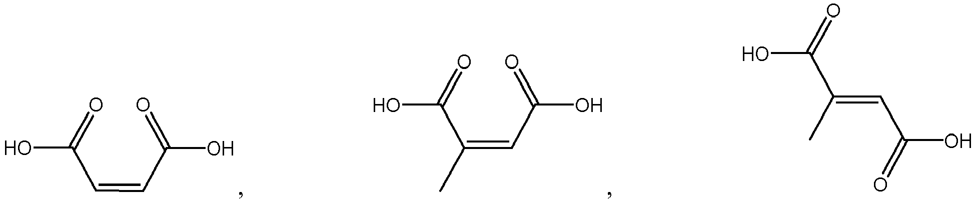 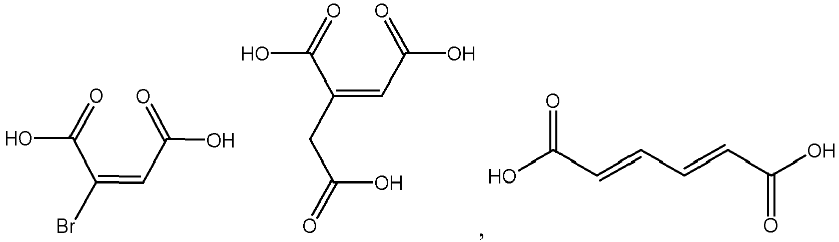 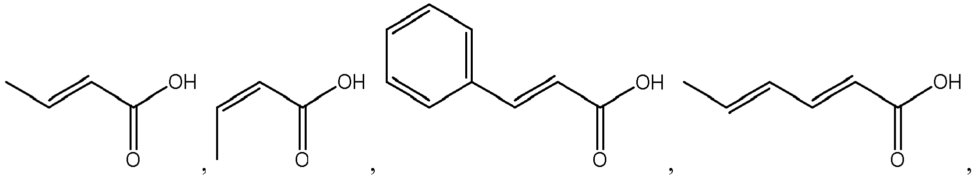 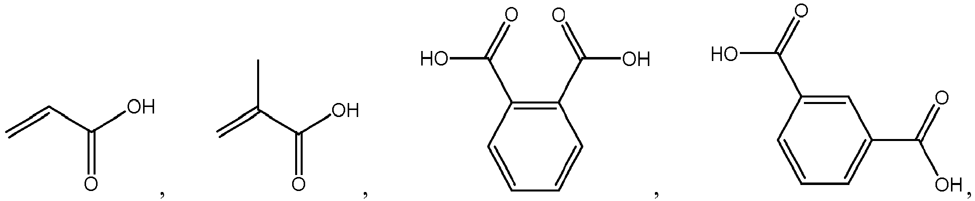 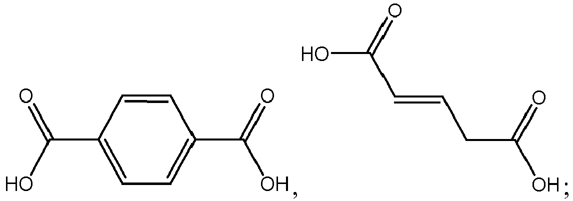 and the cosmetically acceptable salts thereof, and mixtures thereof.

### EXAMPLES

The following examples are non-limiting examples of the hair coloring compositions of the present invention. The examples are given solely for the purpose of illustration, and are not to be construed as limitations of the present invention since many variations thereof are possible without departing from the spirit and scope of the invention, which would be recognized by one of ordinary skill in the art. All concentrations are listed as weight percent (% wt.), unless otherwise specified.

**First composition: Examples 1 to 7**

| **Ingredients** | **Ex.1** (% wt.) | **Ex.2** (% wt.) | **Ex.3** (% wt.) | **Ex.4** (% wt.) | **Ex.5** (% wt.) | **Ex.6** (% wt.) | **Ex.7** (% wt.) |
|---|---|---|---|---|---|---|---|
| *O,O'*-Bis(3-aminopropyl)diethylene glycol | 10.0 | 5.0 | - | - | - | - | - |
| *O,O'*-Bis(2-aminopropyl)ethylene glycol | - | - | 5.0 | 10.0 | - | - | - |
| L-Arginine | - | - | - | - | 5.0 | - | - |
| D-Arginine | - | - | - | - | - | -10 | - |
| L-Lysine | - | - | - | - | - | - | 8.0 |
| Sodium laureth-6 carboxylate | - | - | - | - | - | 2.0 | - |
| Coconut alcohol | - | - | 0.2 | - | - | 0.5 | - |
| Propylene glycol | 0.5 | 0.8 | 1.0 | - | 0.5 | - | - |
| Butylene glycol | - | - | - | - | 0.5 | - | 0.6 |
| PEG-35 Castor oil | 0.3 | 0.3 | - | 0.3 | - | - | - |
| Dimethicone | - | - | - | - | - | - | 0.5 |
| Ceteareth-12 | - | - | - | - | - | 0.5 | - |
| Ceteareth-20 | - | 1.0 | - | - | - | 0.5 | - |
| Ceteareth-25 | 1.0 | - | - | 1.0 | - | - | - |
| Laureth-12 | - | - | - | - | - | - | 1.0 |
| Deceth-3 | - | - | - | - | - | - | 1.0 |
| Oleth-30 | - | - | - | - | - | - | 1.0 |
| Sodium Laureth Sulphate | 2.0 | 2.0 | 1.0 | 2.0 | - | - | - |
| Sodium Lauryl Sulphate | 2.0 | - | 2.0 | 2.0 | - | - | - |
| Disodium EDTA | - | 0.1 | 0.05 | - | 0.1 | - | - |
| Ascorbic acid | 0.2 | 0.2 | 0.15 | 0.2 | 0.3 | 0.2 | 0.2 |
| Sodium sulfite | - | - | - | - | - | 0.5 | 0.5 |
| Ammonium hydroxide | 2.0 | 2.3 | 1.25 | 2.0 | - | 2.0 | 1.5 |
| Perfume | 0.25 | 0.2 | 0.2 | 0.25 | - | 0.2 | 0.1 |
| Water | QSP | QSP | QSP | QSP | QSP | QSP | QSP |
| Total | 100 | 100 | 100 | 100 | 100 | 100 | 100 |

**Second composition: Examples 8 to 14**

| **Ingredients** | **Ex.8** (% wt.) | **Ex.9** (% wt.) | **Ex.10** (% wt.) | **Ex.11** (% wt.) | **Ex.12** (% wt.) | **Ex.13** (% wt.) | **Ex.14** (% wt.) |
|---|---|---|---|---|---|---|---|
| Malic acid | 11.0 | - | - | - | 5.0 | - | - |
| Maleic acid | - | 10.0 | - | - | - | - | - |
| Succinic acid | - | - | 6.0 | - | - | - | - |
| Acotinic acid | | | | 12.0 | | | |
| Pimelic acid | | | | | 5.0 | | |
| Muconic acid | | | | | | 20.0 | |
| Glutaconic acid | | | | | | | 10.0 |
| Cetyl alcohol | 7.0 | 14.0 | - | 7.0 | - | 6.0 | 6.5 |
| Stearyl alcohol | 7.0 | - | 12.0 | 7.0 | - | 6.0 | 6.5 |
| Sodium laureth-6 carboxylate | - | - | - | - | - | 2.0 | - |
| Coconut alcohol | - | - | 0.2 | - | - | 0.5 | - |
| Propylene glycol | - | - | - | - | 25.0 | - | 10.0 |
| Sodium myreth sulphate | - | - | - | - | - | 2.0 | - |
| Glyceryl stearate | 4.0 | 3.5 | 5.0 | 4.0 | - | - | - |
| Glyceryl oleate | 4.0 | 3.5 | - | 4.0 | | - | - |
| PEG-7 Glyceryl Cocoate | 5.0 | 5.5 | - | 5.0 | - | - | 2.0 |
| 2,5-diaminotoluene sulphate | 2.1 | 1.8 | - | 2.1 | - | 1.5 | 1.5 |
| 2,4-diamino phenoxyethanol HCl | - | 0.01 | 2.0 | - | - | - | - |
| 1-methyl-2,5-diamino benzene | - | - | - | - | 1.7 | - | - |
| *N,N*-bis(2-hydroxyethyl)-p-phenylenediamine | - | - | 2.5 | - | - | - | - |
| sulphate | | | | | | | |
| 2-amino-4-hydroxyethylamino anisole sulphate monohydrate | - | - | 1.5 | - | - | - | - |
| 2-methyl-5-hydroxyethyl aminophenol | - | - | 1.0 | - | - | - | - |
| 3-amino-2,6-dimethylphenol | - | - | 2.0 | - | - | - | - |
| 2-amino-5-ethylphenol HCl | - | - | 1.0 | - | - | - | - |
| Hydroxyethyl-3,4-methylenedioxyaniline HCl | - | - | 0.5 | - | - | - | - |
| 1,4-diamino-2-(methoxymethyl)-benzene | - | - | 1.8 | - | 4.0 | - | - |
| Phenyl methyl pyrazolone | - | - | 0.25 | - | - | - | - |
| 1-hydroxyethyl-4,5-diamino pyrazole sulphate | - | - | 3.0 | - | - | 3.0 | 3.0 |
| 4-amino-2-hydroxytoluene | 0.1 | - | 0.5 | 0.1 | 1.4 | 1.0 | 1.0 |
| m-aminophenol | 0.1 | 0.2 | 0.5 | 0.1 | - | 0.1 | - |
| p-aminophenol | - | - | 0.5 | - | 2.5 | - | 0.5 |
| Resorcinol | 1.2 | 0.8 | 1.3 | 1.2 | 0.9 | - | - |
| 2-amino-6-chloro-4-nitrophenol | - | - | 1.0 | - | - | - | - |
| 5-amino-6-chloro-o-cresol | - | - | - | - | - | - | 1.0 |
| 2-methylresorcinol | - | - | 1.0 | - | - | - | - |
| 1-naphthol | - | - | 1.0 | - | - | - | - |
| Lauryl glucoside | - | - | - | - | - | 2.0 | - |
| Decyl glucoside | - | - | - | - | - | - | |
| Acrylamido propyltrimonium chloride/acrylates copolymer | - | - | - | - | - | 1.0 | - |
| Carbomer | - | - | - | - | - | - | 0.5 |
| Hydroxyethylcellulose | - | - | - | - | 1.5 | - | - |
| Polyquaternium-22 | - | - | 0.05 | - | - | - | 0.1 |
| Polyquaternium-10 | 0.3 | 0.3 | - | 0.3 | - | - | - |
| Polyquaternium-7 | 3.0 | 3.0 | - | 3.0 | - | - | - |
| Dimethicone | - | - | - | - | - | - | 0.5 |
| Ceteareth-12 | - | - | - | - | - | 0.5 | - |
| Ceteareth-20 | - | 1.0 | - | - | - | 0.5 | - |
| Ceteareth-25 | 1.0 | - | - | 1.0 | - | - | - |
| Laureth-12 | - | - | - | - | - | - | 1.0 |
| Deceth-3 | - | - | - | - | - | - | 1.0 |
| Oleth-30 | - | - | - | - | - | - | 1.0 |
| Sodium Laureth Sulphate | 2.0 | 2.0 | 1.0 | 2.0 | - | - | - |
| Sodium Lauryl Sulphate | 2.0 | - | 2.0 | 2.0 | - | - | - |
| Macadamia ternifolia seed oil | - | - | - | - | - | 0.1 | - |
| Grape seed oil | - | - | - | - | - | - | 0.1 |
| Sodium sulfite | 0.4 | - | 0.2 | 0.4 | - | 0.2 | - |
| Sodium metabisulfite | - | 0.3 | - | - | 0.7 | - | 0.5 |
| Ammonium sulphate | - | 0.1 | - | - | - | 0.1 | - |
| Sodium sulphate | 0.5 | - | 0.5 | 0.5 | - | - | - |
| Pentasodium pentetate | - | - | - | - | - | - | 0.1 |
| Silica dimethyl silylate (nano) | - | - | - | - | - | - | 0.1 |
| Sodium silicate | - | - | - | - | - | 0.1 | - |
| Etidronic acid | 0.2 | 0.2 | - | 0.2 | - | 0.2 | - |
| Disodium EDTA | - | - | 0.05 | - | 0.1 | - | - |
| Titanium dioxide | - | - | - | - | - | - | 0.1 |
| Monoethanol amine | - | - | - | - | 14.5 | - | 16 |
| Ascorbic acid | 0.2 | 0.2 | 0.15 | 0.2 | 0.3 | 0.2 | 0.2 |
| Lauric acid | - | - | - | - | - | 0.15 | 0.1 |
| Sodium chloride | - | - | - | - | - | 0.1 | 0.1 |
| Potassium hydroxide | - | - | - | - | - | 0.2 | - |
| Sodium hydroxide | 0.03 | - | 0.25 | 0.03 | - | - | - |
| Ammonium hydroxide | 2.0 | 2.3 | 1.25 | 2.0 | - | 2.0 | 1.5 |
| Perfume | 0.25 | 0.2 | 0.2 | 0.25 | - | 0.2 | 0.1 |
| Water | QSP | QSP | QSP | QSP | QSP | QSP | QSP |
| Total | 100 | 100 | 100 | 100 | 100 | 100 | 100 |

The second composition comprising one or more ingredients of Formula II and/or Formula III and a dye composition as exemplified in Ex. 8-14 is mixed with a developer composition at a mixing of 1:1. A suitable developer composition is a commercially available Wella Welloxon™ Perfect Developer Cream, 6% wt. hydrogen peroxide. The resulting mixture is first applied to dried hair strands of hair (3 g per hair strands). After a leave-on time of 30 min at room temperature (23°C), the hair is not rinsed, but dried.

Then, the first composition as exemplified in Exs. 1-7 is applied to the previous treated hair strands of hair (3 g per hair strands) for a leave-on time of 30 min at room temperature (23°C). The hair may be rinsed, washed with a standard shampoo, rinsed and then dried.

Alternatively, the first composition as exemplified in Exs. 1-7 is first applied to hair strands of hair (3 g per hair strands) for a leave-on time of 30 min at room temperature (23°C). The hair may be rinsed, washed with a standard shampoo, rinsed and then dried.

The second composition comprising one or more ingredients of Formula II and/or Formula III and a dye composition as exemplified in Ex. 8-14 is mixed with a developer composition at a mixing of 1:1. A suitable developer composition is a commercially available Wella Welloxon™ Perfect Developer Cream, 6% wt. hydrogen peroxide. The resulting mixture is then applied to hair strands of hair (3 g per hair strands). After a leave-on time of 30 min at room temperature (23°C), the hair is rinsed, washed with a standard shampoo, rinsed and then dried.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A method for coloring hair comprising:
(a) applying to the hair a first composition comprising in a cosmetically acceptable carrier, one or more ionizable linkers of Formula I, or cosmetically acceptable salts thereof, or mixtures thereof: 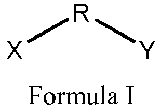
wherein the R group of the one or more ionizable linkers is an alkyl group, alkenyl group, cycloalkyl group, cycloalkenyl group, aryl group, heterocyloalkyl group, or hetereoaryl group;
wherein the alkyl group, alkenyl group, cycloalkyl group, cycloalkenyl group, aryl group, heterocyloalkyl group, or hetereoaryl group is unsubstituted or substituted one or more times by hydrogen, halogen, hydroxy, alkyl, alkenyl, alkynyl, alkoxy, cycloalkyl, cycloalkenyl, cycloalkynyl, aryl, heterocyclyl, heteroaryl, imine, amine, formyl, acyl, carboxylic acid, -C(O)R¹, -C(O)OR¹, (-COO⁻), -CONH₂, -CONHR¹, - C(O)NR¹R², -NR¹R²,-NR¹S(O)₂R², -NR¹C(O)R², -S(O)₂R², -SR¹, -S(O)₂NR¹R², - SOR¹, or -SOOR¹ and mixtures thereof; and
wherein the one or more ionizable linkers comprises two ionizable functional groups X and Y; wherein the one or more ionizable functional groups X, Y are independently selected from the group consisting of: -C(O)NR¹R², -NR¹R²,-NR¹S(O)₂R², - NR¹C(_{O})R²;
wherein R¹ and R² are each independently selected from the group consisting of a hydrogen, alkyl, alkenyl, alkynyl, cycloalkyl, cycloalkenyl, cycloalkynyl, aryl, heterocyclyl, and heteroaryl group;
(b) applying to the hair a second composition comprising in a cosmetically acceptable carrier, one or more ingredients of Formula II, or cosmetically acceptable salts thereof, or mixtures thereof: 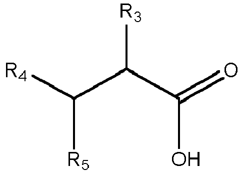 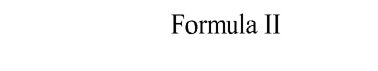
wherein R₃ is independently selected from the group consisting of: -H, -OH, oxo, - H₂N, -C₁₋₆ alkyl groups, -C₁₋₆ alkoxy groups, -C₁₋₆ alkenyl groups;
wherein R₄ is independently selected from the group consisting of: -H, -OH, oxo, - H₂N, -C₁₋₆ alkyl groups, -C₁₋₆ alkoxy groups, -C₁₋₆ alkenyl groups;
wherein R₅ is independently selected from the group consisting of: -H, -C₁₋₆ alkyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid, and ionizable functional groups;
wherein the ionizable functional group is independently selected from the group consisting of: -COOH, -SO₃H, -PO₃H₂; and/or
wherein the second composition comprises in a cosmetically acceptable carrier, one or more ingredients of Formula III, or cosmetically acceptable salts thereof, or mixtures thereof: 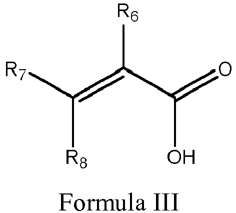
wherein R₆ is independently selected from the group consisting of: -H, -C₁₋₆ alkyl groups, aryl groups, and ionizable functional groups;
wherein R₇ is independently selected from the group consisting of: -H, -C₁₋₆ alkyl groups, aryl groups, and ionizable functional groups;
wherein R₈ is independently selected from the group consisting of: -H, -C₁₋₆ alkyl groups, -C₁₋₆ alkenyl groups, -C₁₋₆ alkyl groups substituted with -C₁₋₆ alkoxycarbonyl group, -C₁₋₆ alkyl groups substituted with -C₁₋₆ alkylcarbamoyl group, -C₁₋₆ alkyl group substituted with a terminal carboxylic acid; and ionizable functional groups;
wherein the ionizable functional group is independently selected from the group consisting of: -COOH, -CH₂COOH, -SO₃H, -PO₃H₂;
and
wherein the first composition does not comprise any ingredients of Formula II and/or Formula III of the second composition and any oxidative dye precursors; wherein the second composition does not comprise any ionizable linkers of Formula I of the first composition; and
wherein the second composition further comprises a dye composition and optionally a developer composition wherein the developer composition comprises one or more oxidizing agents; and wherein the dye composition comprises one or more oxidative dye precursors comprising one or more couplers and one or more primary intermediates.

2. The method according to any preceding claims, wherein step (b) occurs prior to step (a).

3. The method according to any preceding claims wherein the one or more ionizable linkers of Formula I are selected from the group consisting of: 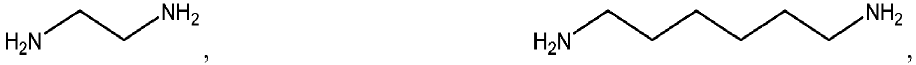 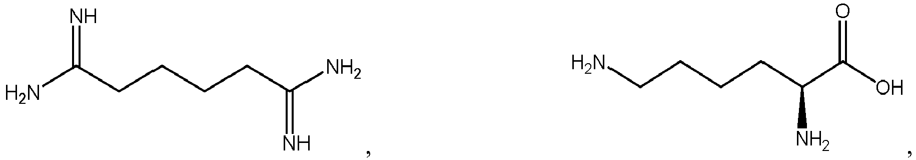 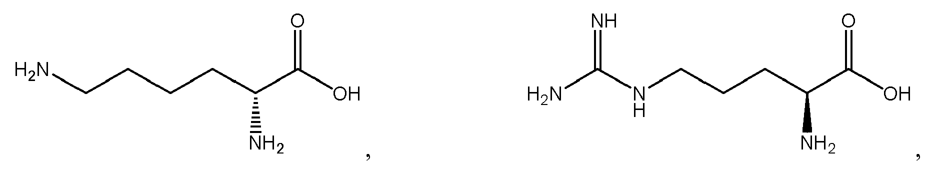 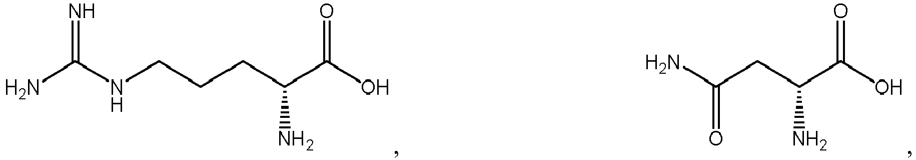 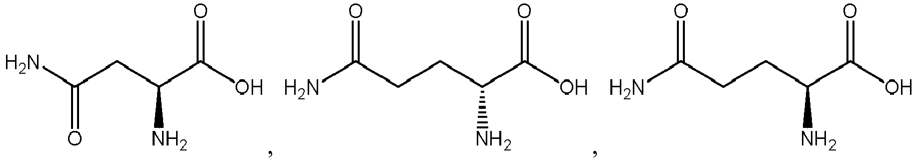 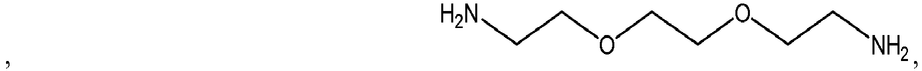 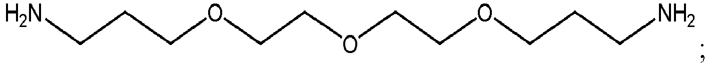 cosmetically acceptable salts thereof, and mixtures thereof.

4. The method according to any preceding claims wherein the one or more ingredients of formula II are selected from the group consisting of acetic acid, glycolic acid, propionic acid, pyruvic acid, lactic acid, glyceric acid, butyric acid, isobutyric acid, valeric acid, isovaleric acid, caproic acid, pivalic acid, malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid, sulfoacetic acid, phosphonoacetic acid; and the cosmetically acceptable salts thereof, and mixtures thereof.

5. The method according to any of the claims 1-3 wherein the one or more ingredients of formula III are selected from the group consisting of: 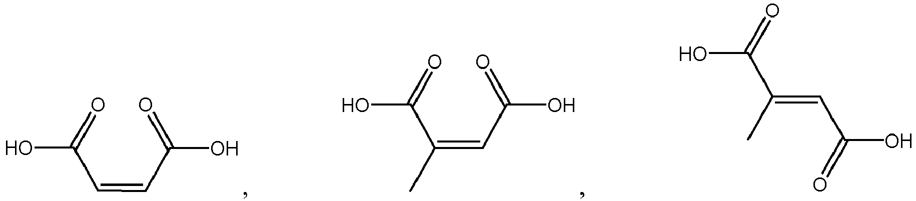 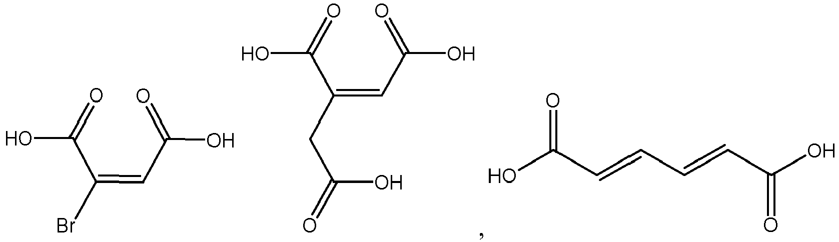 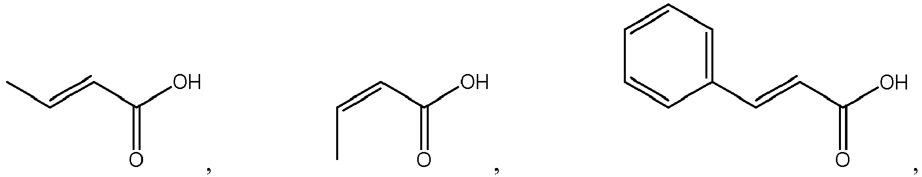 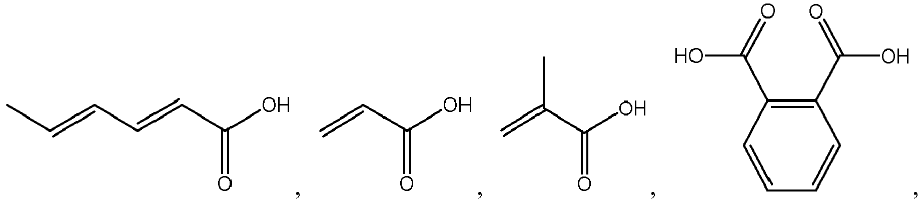 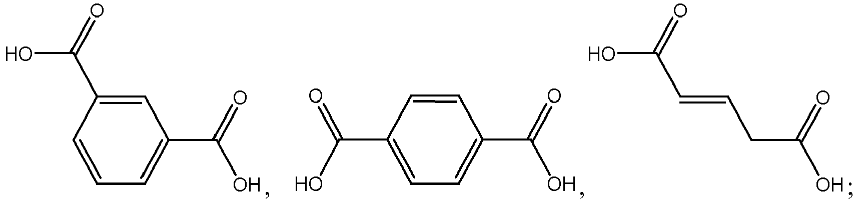 and the cosmetically acceptable salts thereof, and mixtures thereof.

6. The method according to claim 4 wherein the one or more ionizable linkers of Formula I are selected from the group consisting of: 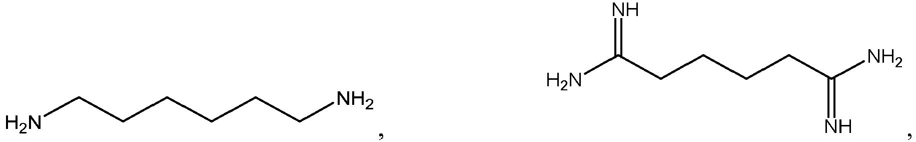 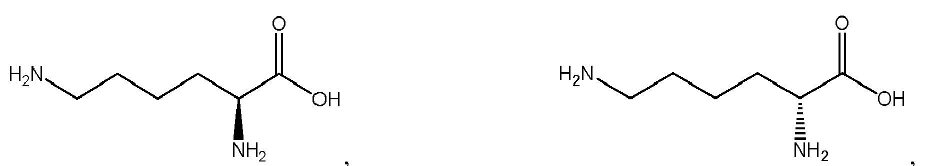 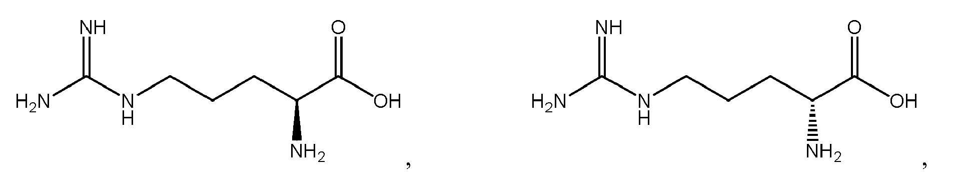 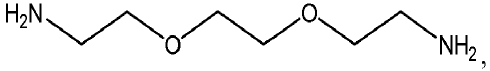 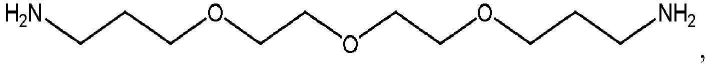 cosmetically acceptable salts thereof, and mixtures thereof; and
wherein the one or more ingredients of Formula II are selected from the group consisting of malonic acid, succinic acid, malic acid, itaconic acid, aspartic acid, glutaric acid, α-ketoglutaric acid, glutamic acid, adipic acid, pimelic acid, suberic acid, azelaic acid, sebacic acid, 1,2,3-propanetricarboxylic acid, citric acid, tartaric acid; and the cosmetically acceptable salts thereof, and mixtures thereof.

7. The method according to claim 5 wherein the one or more ionizable linkers of Formula I are selected from the group consisting of: 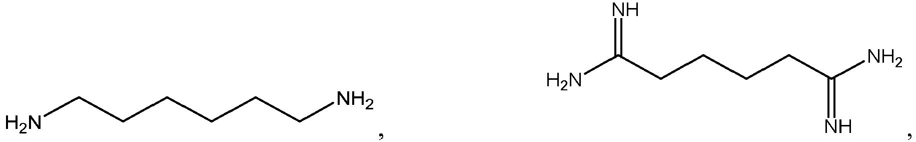 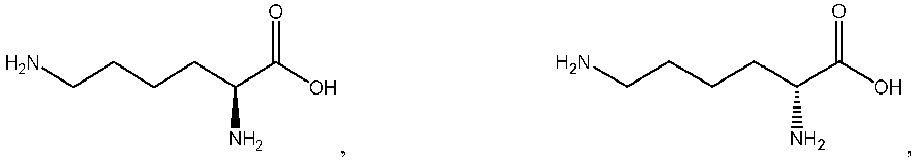 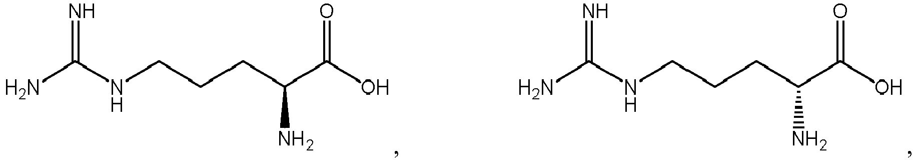 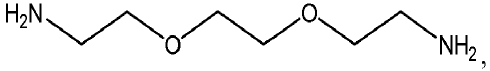 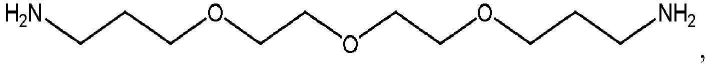 cosmetically acceptable salts thereof, and mixtures thereof; and
wherein the one or more ingredients of Formula III are selected from the group consisting of: 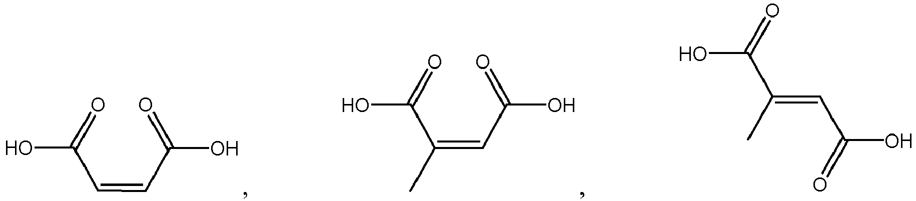 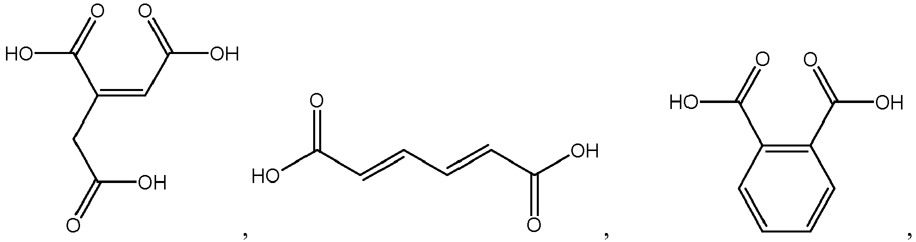 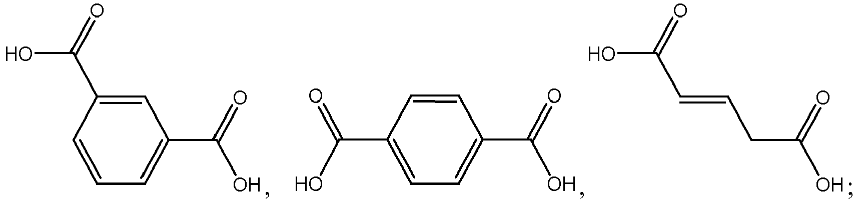 and the cosmetically acceptable salts thereof, and mixtures thereof

8. The method according to any preceding claims, wherein the first composition comprises one or more oxidizing agents.

9. The method according to Claim 8, wherein the first composition is substantially free of oxidizing agent.

10. The method according to any preceding claims, wherein the first composition comprises from 0.1% to 25% of the one or more ionizable linkers by total weight of the first composition.

11. The method according to any preceding claims, wherein the second composition comprises from 0.1% to 25% of the one or more ingredients of Formula II or Formula III by total weight of the second composition.

12. The method according to any preceding claims, wherein the one or more primary intermediates are selected from the group consisting of toluene-2,5-diamine, p-phenylenediamine, N-phenyl-p-phenylenediamine, N,N-bis(2-hydroxyethyl)-p-phenylenediamine, 2-hydroxyethyl-p-phenylenediamine, hydroxypropyl-bis-(N-hydroxyethyl-p-phenylenediamine), 2-methoxymethyl-p-phenylenediamine, 2-(1,2-dihydroxyethyl)-p-phenylenediamine, 2,2'-(2-(4-aminophenylamino)ethylazanediyl)diethanol, 2-(2,5-diamino-4-methoxyphenyl)propane-1,3-diol, 2-(7-amino-2H-benzo[b][1,4]oxazin-4(3H)-yl)ethanol, 2-chloro-p-phenylenediamine, p-aminophenol, p-(methylamino)phenol, 4-amino-m-cresol, 6-amino-m-cresol, 5-ethyl-o-aminophenol, 2-methoxy-p-phenylenediamine, 2,2'-methylenebis-4-aminophenol, 2,4,5,6-tetraminopyrimidine, 2,5,6-triamino-4-pyrimidinol, 1-hydroxyethyl-4,5-diaminopyrazole sulfate, 4,5-diamino-1-methylpyrazole, 4,5-diamino-1-ethylpyrazole, 4,5-diamino-1-isopropylpyrazole, 4,5-diamino-1-butylpyrazole, 4,5-diamino-1-pentylpyrazole, 4,5-diamino-1-benzylpyrazole, 2,3-diamino-6,7-dihydropyrazolo[1,2-a]pyrazol-1(5H)-one dimethosulfonate, 4,5-diamino-1-hexylpyrazole, 4,5-diamino-1-heptylpyrazole, methoxymethyl-1,4-diaminobenzene, N,N-bis(2-hydroxyethyl)-N-(4-aminophenyl)-1,2-diaminothane, 2-[(3-aminopyrazolo[1,5-a]pyridin-2-yl)oxy]ethanol hydrochloride, salts thereof and mixtures thereof.

13. The method according to any preceding claims, further comprising:
(c) rinsing, shampooing, conditioning the hair, or a combination thereof,
wherein step (c) occurs subsequent to step (a) and/or step (b).

14. A kit for coloring hair comprising:
(a) a first composition comprising in a cosmetically acceptable carrier, one or more ionizable linkers of Formula I as defined in any of the claims 1-13;
(b) a second composition comprising in a cosmetically acceptable carrier, one or more ingredients of Formula II and/or Formula III as defined in any of the claims 1-13;
wherein the first composition does not comprise any ingredients of Formula II and/or III of the second composition and any oxidative dye precursors;
wherein the second composition does not comprise any ionizable linkers of Formula I of the first composition; and
(c) a dye composition comprising one or more oxidative dye precursors;
(d) optionally, a developer composition comprising one or more oxidizing agents;
wherein the first composition, the second composition, the dye composition (c) and optionally the developer composition (d) are separately packaged in different containers or packaged in a same container in different compartments; and
(e) an instructional material for preparation, for use, or both preparation and use, of the first composition, the second composition, the dye composition and optionally the developer composition.

15. The kit according to Claim 13, wherein the dye composition is mixed with the second composition, optionally with the developer composition.
